(19) Europäisches Patentamt
European Patent Office
Office européen des brevets

(11) **EP 4 150 340 B1**

(12) **EUROPEAN PATENT SPECIFICATION**

(45) Date of publication and mention
of the grant of the patent:
**08.11.2023 Bulletin 2023/45**

(21) Application number: **21722932.7**

(22) Date of filing: **06.05.2021**

(51) International Patent Classification (IPC):
*G01N 33/543* (2006.01)    *A61B 5/145* (2006.01)
*A61B 5/1495* (2006.01)    *A61B 5/00* (2006.01)

(52) Cooperative Patent Classification (CPC):
**G01N 33/54306; A61B 5/14507; A61B 5/14517;**
**A61B 5/1495; A61B 5/4266; G01N 33/54366;**
**G01N 33/5438**

(86) International application number:
**PCT/EP2021/062039**

(87) International publication number:
**WO 2021/228688 (18.11.2021 Gazette 2021/46)**

(54) **CALIBRATION USING A REGENERATIVE SURFACE**

KALIBRIERUNG UNTER VERWENDUNG EINER REGENERATIVEN OBERFLÄCHE

ÉTALONNAGE À L'AIDE D'UNE SURFACE RÉGÉNÉRATIVE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.05.2020 EP 20173942**

(43) Date of publication of application:
**22.03.2023 Bulletin 2023/12**

(73) Proprietor: **Koninklijke Philips N.V.**
**5656 AG Eindhoven (NL)**

(72) Inventors:
• **VAN LIESHOUT, Ron, Martinus, Laurentius**
**5656 AE Eindhoven (NL)**

• **JOHNSON, Mark, Thomas**
**5656 AE Eindhoven (NL)**
• **DELLIMORE, Kiran, Hamilton, J.**
**5656 AE Eindhoven (NL)**
• **HUIJBREGTS, Laurentia, Johanna**
**5656 AE Eindhoven (NL)**

(74) Representative: **Philips Intellectual Property &
Standards**
**High Tech Campus 52**
**5656 AG Eindhoven (NL)**

(56) References cited:
**EP-A1- 3 646 788       WO-A1-01/67079**
**US-A1- 2015 260 676    US-A1- 2019 142 311**
**US-A1- 2020 070 161**

**Description**

FIELD OF THE INVENTION

The present invention relates to body fluid monitoring, and in particular to a calibration unit, a body fluid monitoring apparatus, a method for calibrating a sensor device, and a computer program element for calibrating a sensor device.

BACKGROUND OF THE INVENTION

Sensors, such as biosensors and electrochemical sensors, are known to drift in time and require calibration or recalibration. For electrochemical sensors the drift is caused by reduction of functionality (e.g. enzyme functionality), inherent drift and drift caused by fouling of the sensor.

Typically, the calibration is done using a calibration liquid with a known concentration of the molecule of interest. The calibration liquid can be stored inside the patch, but this solution may cause stability, patch design and re-use problems. Another way is to administer the calibration liquid manually to the patch, but this solution requires trained personnel to perform the liquid administration, increasing the calibration process complexity and the likelihood of errors. A third option is calibration using a standardized measurement method, e.g. a central lab test; again this option has its drawbacks causing additional burden to the patient, it requires trained personnel and may introduce workflow issues.

Moreover, matrix effects are a big issue in the biosensor area. The matrix effect, i.e. change of binding conditions due to interfering components in the bioliquid, is caused by the presence of other molecules than the ones of interest in the same bioliquid and lead to an over- or underestimation of the true examined concentration. The matrix effect differs from patient to patient and when the patient has an illness, it can be increased.

US 2019/142311 A1 describes devices for calibrating electrochemical aptamer-based biofluid sensors. In some embodiments a calibrant is located proximate to the EAB sensors, and calibration is facilitated by introduction of the biofluid into the device. Other embodiments include various mechanisms for introducing calibration solution to the sensors, including drawing calibration solution into contact with sensors through electrical potential, a dispensing mechanism such as an ink jet-type nozzle, as well as pressure actuated calibration dispensing. Also included are embodiments employing bifurcated biofluid transport paths that allow alternately for calibration and biofluid sensing.

SUMMARY OF THE INVENTION

There may be a need to improve biosensor calibration.

The present invention is defined by the independent claims, wherein further embodiments are incorporated in the dependent claims. It should be noted that the following described aspects of the invention apply also for the calibration unit, the body fluid monitoring apparatus, and the computer program element.

According to a first aspect of the present invention, there is provided a calibration unit adapted for calibrating a sensor device adapted for detecting analyte molecules in a fluid sample of a subject. The calibration unit comprises a calibration matrix having a capture surface with calibration receptors being immobilized thereon adapted for reversibly binding analyte molecules in a fluid sample of the subject. The bound analyte molecules represent calibration molecules usable for calibrating the sensor device. The calibration unit further comprises a regeneration assembly configured to regenerate the capture surface by releasing the calibration molecules from the capture surface into an aqueous solution to form a calibration fluid for calibrating the sensor device.

In other words, it is proposed to use a reagent free calibration method for calibrating a sensor device. The reagent free calibration method comprises capturing analyte molecules (i.e. calibration molecules) inside the bioliquid of the patient and releasing them when needed for calibration. Accordingly, this reagent free calibration method may eliminate the need for on-board reagent storage. The proposed calibration unit is useful for any biosensor that uses calibration methods and is in prolonged contact with the bioliquid of interest. Examples of the biosensors may include, e.g. wearables, patches, insertables, implantables. Examples of the biosensors may also include sensor elements inside the bioliquid of interest for performing measurements e.g. in the interstitial fluid and/or in blood, for example, via a central line.

To this end, a calibration unit is proposed that comprises a calibration matrix having a capture surface with calibration receptors being immobilized thereon for binding analyte molecules in a fluid sample of the subject. Optionally, the calibration unit may be an integrated part of the sensor device. The calibration receptors refer to the receptors in the calibration unit. The receptor may refer to any molecule, either synthetic or natural in origin, which has reasonable affinity and specificity for one or more analyte molecules. The immobilized receptors and analyte molecules may also be referred to as biological binding partners, which may include the variety of known ligands. For example, one skilled in the art will appreciate that the biological binding partners may include e.g. an antigen or antibody, a hormone or neurotransmitter and a receptor, a substrate or allosteric effector and an enzyme, lectins and sugars, DNA or RNA structures, such as aptamers and their binding species (including other DNA or RNA species or binding protein), proteins, biotin and adivin or streptavidin systems, enzymes and their substrates and inhibitors, lipid binding systems, and combinations thereof. The binding of the analyte molecules and the receptors forms receptor-analyte complexes. For example, antibodies may be immobilized on the capture surface of the calibration unit, which antibodies capture a particular antigen; the antigen being the analyte of interest (i.e. analyte molecules). Thus, when a sample contacts the surface, the calibration receptors may bind the analyte present in the sample.

With the calibration receptors, the capture surface of the calibration unit can capture a sufficient amount of analyte molecules. The amount of analyte molecules captured by the capture surface can be controlled by choosing a predetermined amount of calibration receptors on the capture surface and the type of receptors molecules. When the pre-defined amount of analyte molecules is captured, they are ready to be used as calibration molecules, which can be released into an aqueous solution, such as buffered solution or fluid sample of the subject, to form a calibration fluid. The reversible nature of the binding of the analyte to the calibration receptors means that the analyte may in principle be liberated from the calibration receptors, thereby to regenerate the sensor surface to form a calibration fluid and to allow for re-use of the calibration area.

In order to regenerate the capture surface, the calibration unit further comprises a regeneration assembly. In the following, three examples of the regeneration assembly will be briefly described.

The first example of the regeneration assembly is based on the realization that electrolysis of the aqueous medium may be employed to regenerate the capture surface. To this end, a regeneration assembly is proposed that comprises an electrolysis assembly for electrolysing the aqueous solution, e.g. the fluid sample or the buffered solution. A voltage, e.g. a voltage profile, is supplied across pairwise combinations of a plurality of spatially separated conductive areas on the surface; the respective conductive areas of each pairwise combination thus becoming an anode and a cathode. The voltage is sufficient to electrolyse the aqueous medium, which results in hydrogen ions being produced at the anode, and hydroxyl ions being produced at the cathode. The pH local to the anode may thus be lowered, and the pH local to the cathode may be increased. These localized changes to the pH may result in disruption, e.g. denaturation, of the receptor-analyte complex, such as to regenerate at least part of the capture surface.

The second example of the regeneration assembly is based on the realization that an electrical potential may be generated in response to the flow of fluid through the calibration unit. To this end, a regeneration assembly is proposed that may comprise: a flow channel arranged such that fluid flows through the flow channel; and an induction electrode (or array) arranged circumferentially around the periphery of the flow channel. The induction electrode (or array) may generate a triboelectric potential due to moving ions in the fluid flowing through the flow channel. The triboelectric potential generated by the induction electrode may correspond to the rate of moving ions in the fluid flowing through the flow channel. More specifically, to the velocity and concentration (i.e. flow rate and concentration rate) of moving ions in the fluid flowing through the flow channel. The ions moving through the flow channel may interact with the induction electrode to induce a triboelectric potential (voltage), which may be used directly as the current for the regeneration capture surface.

Alternatively or additionally, the calibration unit may comprise: a flow channel arranged such that fluid flows through the flow channel; and one or more ion-selective electrodes arranged inside the flow channel. The ion-selective electrode may generate an electrochemical potential due to moving ions in the fluid flowing through the flow channel. The electrochemical potential generated by the ion-selective electrode may correspond to the rate or concentration of (specific) moving ions in the fluid flowing through the flow channel. The ions moving through the flow channel may interact with the ion-selective electrode to induce an electrochemical potential (voltage), which may be used directly as the current for the regeneration capture surface.

The third example of the regeneration assembly is based on the realization that the pH of the first fraction of droplets of a fluid sample (e.g. sweat, sebum, and interstitial fluid) excreted on the skin surface is initially around 2, which sweat, sebum, and interstitial fluid is sufficiently acidic to support the release of the calibration molecules from the capture surface. In case of sweat droplets, for example, as the sweat rate increases, the pH rises gradually until it reaches a near neutral pH, i.e. pH~7. This occurs due to the inverse relation between the reabsorption flux of ions along the sweat duct and the sweat rate. Therefore, the first fraction of sweat droplet or droplets emerging on the skin surface form an active sweat gland will have a pH of 2 and only this first fraction is used to release the analyte molecules. To this end, the regeneration assembly may have a chamber which collects a fluid sample from the skin via an inlet. The chamber also has an outlet which is arranged, e.g. dimensioned, such that a droplet of the fluid sample protrudes therefrom once the chamber has been filled. A fluid transport assembly releases the droplet fluid sample from the outlet, and transports the released droplet towards the sensor. The fluid transport assembly retains the discrete form of the droplet as the droplet is being transported towards the sensor. Dropwise supply of the droplets to the sensor is provided due to the transportation of the released droplet being at least as fast as, and preferably faster than, the protrusion of a subsequent droplet of the fluid sample from the outlet. This avoids that such a subsequent sweat droplet protrudes against, and coalesces with, a released sweat droplet which is in the process of being transported to the sensor. In particular, the first fraction of a droplet or droplets of the fluid sample (e.g. sweat, sebum, and interstitial fluid) emerging on the skin surface having a pH of 2 will be transport to the capture surface by means of an electromechanical (e.g. electrowetting) or chemical gradient. These localized changes to the pH may result in disruption, e.g. denaturation, of the receptor-analyte complex, such as to regenerate at least part of the capture surface.

In an example, the calibration unit may be in the same transport channel as the sensor device. In another example, the calibration unit may be arranged in a transport channel different from the sensor device. In this example, the capture area of the calibration unit may be pre-loaded and the liquid may be removed from the capture area by using a capillary pump and evaporator, such that the calibration molecules are less prone to degenerated. When calibration is needed, valves and the regeneration assembly are configured to release the calibration molecules.

Typically, calibration is done using a calibration curve/range. In an example, by integrating multiple capture areas into the calibration unit and releasing them at different time intervals, a calibration curve can be made to calibrate the sensor. The time separated release of calibration molecules may be done by:

(1) activating the regeneration zone at different time intervals resulting in different concentration;
(2) having multiple zones with different amount of receptors (i.e. size of capture area) that provide different concentrations after release; and/or
(3) having receptors that release at different pH values. By incorporating a mix of these receptor types one could trigger a certain concentration by activating the same regeneration zone with different pH conditions. Note that antibodies can be designed to have different $K_{off}$ related to the pH conditions.

According to an embodiment of the present invention, the aqueous solution is a fluid sample of the subject.

Because the calibration molecules are in the same matrix (i.e. bioliquid) as the measurement the matrix effects (i.e. change of binding conditions due to interfering components in the bioliquid) is also corrected for. A normal calibration liquid is a buffered solution and is per definition not the same liquid as the bioliquid, i.e. the fluid sample of the same subject.

According to an embodiment of the present invention, the regeneration assembly is configured to apply an electrical potential to the capture surface to release the calibration molecules from the capture surface.

According to an embodiment of the present invention, the regeneration assembly comprises an electrolysis assembly configured to electrolyse the aqueous solution.

In this example, the regeneration assembly is based on the realization that water electrolysis may be employed to regenerate the surface, such as to remove or minimize the requirement for reagents to effect regeneration. The proposed regeneration assembly comprises an electrolysis assembly for electrolysing the aqueous medium. A voltage is supplied across at least two of a plurality of spatially separated conductive areas on the surface; the at least two conductive areas thus becoming an anode and a cathode. The voltage is sufficient to electrolyse the water, which results in hydrogen ions being produced at the anode, and hydroxyl ions being produced at the cathode. The pH local to the anode may thus be lowered, and the pH local to the cathode may be increased. These localized changes to the pH may result in disruption, e.g. denaturation, of the capture species-analyte complex, such as to regenerate at least part of the surface.

According to an embodiment of the present invention, the electrolysis assembly comprises at least three spatially separated electrically conductive areas on the capture surface and a power supply. The power supply is configured to implement a first setting in which a voltage sufficient to electrolyse the aqueous solution received on the capture surface is supplied across a first pairwise combination of said at least three conductive areas and a second setting in which a voltage sufficient to electrolyse the aqueous solution received on the capture surface is supplied across a second pairwise combination of said at least three conductive areas, the second pairwise combination being different from said first pairwise combination.

The respective pairwise combinations may, for example, be different from each other in terms of both the spacing between the conductive areas and the arrangement of voltage polarity applied to the (alternating) conductive areas.

The voltage may be different in the first and second configurations. For example, the voltage may be higher or linearly increasing in the second setting than in the first setting. Alternatively or additionally, the second pairwise combination of conductive areas may be spaced further apart on the surface than the first pairwise combination of conductive areas. Such measures may assist to increase the degree of electrolytic regeneration of the capture surface.

The conductive areas may be arranged relative to each other in an interdigitated configuration, e.g. in a planar interdigitated configuration. The interdigitated configuration may assist to provide the voltage over a relatively large area, e.g. in the order of several $mm^2$ to $cm^2$, whilst providing localities on the surface in which the local pH changes resulting from water electrolysis drive regeneration. A similar effect may be achieved using, for instance, electrically conductive areas arranged as a plurality of concentric ring-like portions.

The plurality of spatially separated electrically conductive areas may comprise an array of conductive areas in which the spacing between adjacent conductive areas alternates between a larger and a smaller separation, wherein the power supply may be configured to supply the voltage across adjacent conductive areas spaced by the larger separation, the adjacent conductive areas spaced by the smaller separation being of the same polarity as each other.

Such an arrangement may assist to reduce the area on the surface in which electrolytic regeneration does not occur. Accordingly, the degree of electrolytic regeneration of the surface may be enhanced.

The plurality of spatially separated electrically conductive areas may comprise a grid of electrically conductive strips, the power supply being configured to implement: a first mode in which the voltage (profile) is supplied across parallel strips extending in a first direction; and a second mode in which the voltage is supplied across parallel strips extending in a second direction, the second direction being different from the first direction. The power supply may, for instance, be configured to implement the first and second modes sequentially. More complete regeneration of the surface may thus be achieved.

The power supply may be configured to switch the polarity of the voltage across the at least two electrically conductive areas. Certain receptor-analyte (e.g. antibody-antigen) complexes, for instance, may be denatured only at an optimum acidic or alkaline pH, and are otherwise robust to large pH variations. For this reason, the power supply may be configured to switch the polarity of the voltage across the respective electrically conductive areas or superimpose a larger AC voltage to a DC voltage to switch the polarity of the electrodes and direction of the electric field. This polarity switch may result in regeneration of capture species located proximal to both of the respective conductive areas.

According to an embodiment of the present invention, the calibration unit further comprises an assembly that comprises a flow channel arranged such that the aqueous solution flows through the flow channel. The assembly further comprises an electrode arrangement that comprises an induction electrode and/or an ion-selective electrode. The induction electrode is arranged circumferentially around a periphery of the flow channel and configured to generate a triboelectric potential in response to moving ions in the aqueous solution flowing through the flow channel. The ion-selective electrode is arranged inside the flow channel and configured to generate an electrochemical potential in response to moving ions in the aqueous solution flowing through the flow channel.

In other words, an electric potential may be generated by the calibration unit directly from the detection of fluid. That is, the detection of fluid may generate a voltage which may be used to trigger the activation of the regeneration assembly or used directly as the current for the regenerable capture surface of the calibration unit.

The first example of the assembly may comprise: a flow channel arranged such that fluid flows through the flow channel; and an induction electrode (or array) arranged circumferentially around the periphery of the flow channel. The induction electrode (or array) may generate a triboelectric potential due to moving ions in the fluid flowing through the flow channel. The triboelectric potential generated by the induction electrode may correspond to the rate of moving ions in the fluid flowing through the flow channel. More specifically, to the velocity and concentration (flow rate and concentration rate) of moving ions in the fluid flowing through the flow channel. The ions moving through the flow channel may interact with the induction electrode to induce a triboelectric potential (voltage).

The induction electrode may, for example, be arranged in series as circumferential elements/arrays of the flow channel such that, if a plurality of induction electrodes is provided, the fluid flows between a pair of induction electrodes. The induction electrode may be measured against ground. There may preferably be provided at least two induction electrodes: a positive electrode and a grounded electrode or reference electrode. The triboelectric potential generated at the induction electrodes may correspond to the differential between the two electrodes and may provide a passive trigger of calibration molecules release.

The second example of the assembly may comprise: a flow channel arranged such that fluid flows through the flow channel; and one or more ion-selective electrodes arranged inside the flow channel. The ion-selective electrode may generate an electrochemical potential due to moving ions in the fluid flowing through the flow channel. The electrochemical potential generated by the ion-selective electrode may correspond to the rate or concentration of (specific) moving ions in the fluid flowing through the flow channel. The ions moving through the flow channel may interact with the ion-selective electrode to induce an electrochemical potential (i.e. voltage).

The ion-selective electrode and reference electrode may be arranged, e.g. concentrically arranged, in the flow channel such that the fluid flows around the reference electrode. There may preferably be provided at least two ion-selective electrodes: a positive electrode and a grounded electrode or reference electrode. The generated signal of the ion-selective electrode may be measured against the (potential on the) reference electrode. The fluid may flow through the ion-selective membrane electrode and the electrode may be comparable to a potentiometric pH measurement probe using a reference potential/solution.

According to an embodiment of the present invention, the assembly is (i) a regeneration assembly configured to apply at least one of the triboelectric potential and the electrochemical potential to the capture surface to release the calibration molecules from the capture surface; or (ii) a trigger assembly configured to trigger the activation of the regeneration assembly when at least one of the generated triboelectric potential and the generated electrochemical potential exceeds a predetermined threshold corresponding to a given rate of moving ions.

In other words, the transient or pulsatile sweat flow wave (moving ions) through the microfluidic system generates a liquid triboelectric or electrochemical potential that can be used to trigger the activation of the regeneration assembly or used directly as the current for the regenerable capture surface.

According to an embodiment of the present invention, the regeneration assembly comprises a fluid collection assembly. The fluid collection assembly comprises a chamber having an inlet for receiving a droplet of a fluid sample excreted on a skin surface, and an outlet arranged such that the droplet of the fluid sample forms and protrudes therefrom following filling of the chamber with the fluid sample. The regeneration assembly further comprises a fluid transport assembly configured to release the droplet protruding from the outlet and transport the released droplet to the calibration matrix, thereby making the outlet available for a subsequent droplet to form and protrude therefrom upon further filing of the chamber. The fluid transport assembly is arranged to transport the released droplet at least as fast as the subsequent droplet protrudes from the outlet such that the respective droplets do not contact each other. The fluid transport assembly is configured to transport one or more droplets initially excreted on the skin surface to the calibration matrix to release the calibration molecules from the capture surface to form the calibration fluid.

In this example, the regeneration assembly is based on the realization that collecting a fluid sample (e.g. sweat, sebum, and interstitial fluid) excreted on the skin surface and supplying it to the calibration unit in the form of discrete droplets has advantages over employing a continuous flow of the fluid sample. This discretized approach allows the transportation of first fraction of a droplet or droplets of a fluid sample emerging from the skin surface to the capture surface of the calibration unit e.g. by means of an electromechanical or chemical gradient. As the pH of the first fraction of the droplet or droplets is initially around 2, this first fraction of the droplet or droplets may result in disruption, e.g. denaturation, of the receptor-analyte complex, such as to regenerate at least part of the capture surface.

According to an embodiment of the present invention, the calibration matrix is configured to release a different amount of calibration molecules into the aqueous solution to form calibration fluids with different concentrations to construct a calibration curve to calibrate the sensor device.

According to an embodiment of the present invention, the calibration fluids with different concentrations are formed by at least one of: activating the regeneration assembly at different time intervals to release the calibration molecules from the capture surface, thereby resulting in different concentrations; providing multiple capture areas, each of which having a different amount of calibration receptors such that each capture area release a different amount of calibration molecules; and having different types of calibration receptors, each of which configured to release the calibration molecules at a different amount of hydrogen ions.

According to an embodiment of the present invention, the calibration unit further comprises a capillary pump configured to remove a fluid from the capture area and an evaporator configured to evaporate the removed fluid.

In this example, the capture area of the calibration unit may be arranged in a transport channel different from the sensor device. The capture area of the calibration unit may be pre-loaded and the liquid may be removed from the capture area by using a capillary pump and evaporator, such that the calibration molecules are less prone to degenerated. When calibration is needed, valves and the regeneration assembly are configured to release the calibration molecules.

According to a second aspect of the present invention, there is provided a body fluid monitoring apparatus for detecting an analyte in a fluid sample of a subject. The body fluid monitoring apparatus comprises: a calibration unit according to the first aspect and any associated example, a sensor device having a capture surface with sensor molecules being immobilized thereon for detecting the analyte in the fluid sample, wherein the sensor device is in fluid communication with the calibration unit at least in a calibration event, and a fluid collection assembly for supplying the fluid sample to the calibration unit and the sensor device.

According to an embodiment of the present invention, the body fluid monitoring apparatus further comprises a transport channel arranged to accommodate both the calibration unit and the sensor device. Alternatively, the body fluid monitoring apparatus further comprises two transport channels arranged to separately accommodate the calibration unit and the sensor device and a valve arrangement configured to control the flow of a fluid between the two transport channels.

According to a third aspect of the present invention, there is provided an in vitro method for calibrating a sensor device for detecting an analyte in a fluid sample of a subject. The method comprises the following steps:

a) receiving, with a calibration unit, a fluid sample of the subject;
wherein the calibration unit comprises a calibration matrix having a capture surface with calibration receptors being immobilized thereon;
b) reversibly binding, with the calibration receptors, a known amount of analyte molecules in the fluid sample of the subject, wherein the bound analyte molecules represent calibration molecules usable for calibrating the sensor device;
c) regenerating the capture surface, with a regeneration assembly, by releasing the calibration molecules from the capture surface into an aqueous solution to form a calibration fluid; and
d) transporting the released calibration molecules to the sensor device for calibrating the sensor device.

According to another aspect of the present invention, there is provided a computer program element for calibrating a sensor device for detecting an analyte in a fluid sample of a subject, which computer program element, when being executed by a processor, causes the processor to carry out the method according to the third aspect and any associated example.

In this detailed description of the present disclosure, a person skilled in the art should note that directional terms, such as "above," "below," "upper," "lower," and other like terms are used for the convenience of the reader in reference to the drawings. Also, a person skilled in the art should notice this description may contain other terminology to convey position, orientation, and direction without departing from the principles of the present disclosure.

When a fluid sample in any form (e.g., a droplet or a continuous body, whether moving or stationary) is described as being "on", "at", or "over" an electrode, array, matrix or surface, such fluid could be either in direct contact with the electrode/array/matrix/surface, or could be in contact with one or more layers or films that are interposed between the liquid and the electrode/array/matrix/surface.

The term "controller" is used generally to describe various apparatus relating to the operation of a stream probe apparatus, system, or method. A controller can be implemented in numerous ways (e.g., such as with dedicated hardware) to perform various functions discussed herein. A "processor" is one example of a controller which employs one or more microprocessors that may be programmed using software (e.g., microcode) to perform various functions discussed herein. A controller may be implemented with or without employing a processor, and also may be implemented as a combination of dedicated hardware to perform some functions and a processor (e.g., one or more programmed microprocessors and associated circuitry) to perform other functions. Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media (generically referred to herein as "memory," e.g., volatile and non-volatile computer memory). In some implementations, the storage media may be encoded with one or more programs that, when executed on one or more processors and/or controllers, perform at least some of the functions discussed herein. Various storage media may be fixed within a processor or controller or may be transportable, such that the one or more programs stored thereon can be loaded into a processor or controller so as to implement various aspects of the present disclosure discussed herein. The terms "program" or "computer program" are used herein in a generic sense to refer to any type of computer code (e.g., software or microcode) that can be employed to program one or more processors or controllers.

BRIEF DESCRIPTION OF THE DRAWINGS

In the drawings, like reference characters generally refer to the same parts throughout the different views.

Fig. 1 schematically illustrates an example of a calibration unit.
Fig. 2 illustrates a flowchart of a calibration method.
Fig. 3 schematically illustrates the calibration method of Fig. 2 in more detail.
Fig. 4 schematically illustrates an example of a regeneration assembly.
Fig. 5 schematically illustrates exemplary regeneration of the capture surface of the calibration unit.
Fig. 6 schematically shows exemplary interdigitated electrically conductive areas
Fig. 7 schematically shows another exemplary interdigitated electrically conductive area.
Fig. 8 schematically illustrates another example of a regeneration assembly.
Fig. 9 schematically illustrates a cross-section of exemplary induction electrodes.
Fig. 10 shows sweat rate and pH values during a 60-minute effort at self-selected pace.
Fig. 11 schematically illustrates a further example of a regeneration assembly.
Fig. 12 schematically shows an example of a body fluid monitoring apparatus.
Fig. 13 schematically shows a further example of a body fluid monitoring apparatus.

DETAILED DESCRIPTION OF EMBODIMENTS

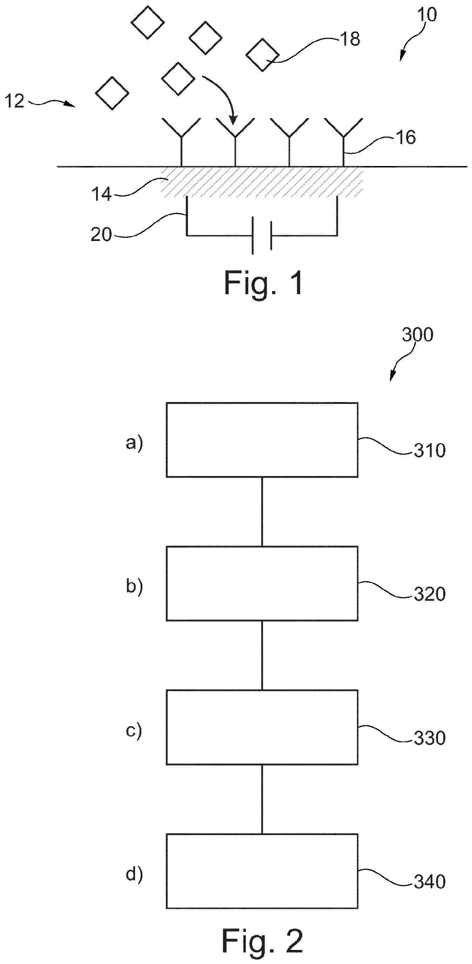

Fig. 1 schematically illustrates an example of a calibration unit 10. The calibration unit 10 comprises a calibration matrix 12 and a regeneration assembly 20.

The calibration matrix 12 having a capture surface 14 with calibration receptors 16 being immobilized thereon for binding a known amount of analyte molecules 18 in a fluid sample of the subject. The fluid sample of the subject may also be referred to as bioliquid, which may include e.g. sweat, sebum, interstitial fluid, blood, urine, or saliva. The bound analyte molecules represent calibration molecules usable for calibrating the sensor device.

The calibration receptors 16 may be selected according to the particular analyte molecules 18, which the sensor device is intended to sense. Relevant analytes molecules 18 may include, but are not limited to, small-molecule compounds, e.g. having a molecular weight less than 900 g/mol, such as urea, creatinine, cholesterol, triglycerides, steroid hormones, e.g. cortisol, glucose and melatonin.

The calibration receptors 16 may be selected to bind other molecule types, such as peptides and proteins. For a protein, the calibration receptors 16 may reversibly bind via an epitope of the protein for which the calibration receptors 16 include a suitable binding site. The calibration receptors 16 may, for example, be selected to bind cytokines such as IL-1alpha, IL-1beta, IL-6, TNF-alpha, IL-8 and TGF-beta IL-6, Cysteine proteinases. Protein binding may, for instance, enable binding and detection of relatively large biomarkers, such as viruses, to the capture surface 14. This is particularly relevant to calibrating a sensor device for sweat sensing because certain viruses, such as the hepatitis C virus, may replicate in sweat glands and be released in sweat.

The calibration receptors 16 may, for instance, include aptamers, which are oligonucleotide or peptide molecules that bind to a specific target molecule. As in the case of antibodies they rely on their three-dimensional configuration in order to attain the requisite affinity for the analyte molecules 18.

In other examples, the calibration receptors 16 may include molecular imprint polymers, which are polymers that are imprinted with an antigen, or part of an antigen. Such materials may comprise a cavity that has a three-dimensional configuration which complements the three-dimensional shape of the antigen, so as to attain the requisite 'lock and key' structure. Molecular imprint polymers may change their structure due to pH changes. One example is a pH sensitive pantoprazole-imprinted polymer, which is known to bind or release a drug according to the pH.

The calibration receptors 16 may be immobilized on the capture surface 14 in any suitable manner. In a non-limiting example, a suitable mercaptan linker may be used to tether the calibration receptors to a capture surface 14 being a gold surface; the linker being grafted to the capture surface 14 via a sulphur-gold interaction and the calibration receptors 16 being grafted, e.g. covalently bonded, to the linker. Numerous alternative means of immobilizing the calibration receptors 16 on the capture surface 14 will be immediately apparent to the skilled person.

In a non-limiting example, the analyte molecules 18 may include an antigen and the calibration receptors may include an antibody. Immunoglobulin (IgG) may, for instance, be a suitable antibody for immobilizing on the capture surface 14. IgG is the most common type of antibody in the human circulation system. Antibodies may be used to capture a wide range of molecules. The antibody structure generally comprises a constant domain (Fc) and an antigen binding domain (Fab), but other forms containing, for instance, only the Fab part, or a portion of the Fab part, may be used. The antigen may include, for example, proteins and polysaccharides. As is well-known per se, antibodies comprise proteins that are produced to fight foreign intruding species, such as viruses. A so-called 'lock and key' interaction selectively binds the antigen to the antibody. The strength of the binding between the antibody and the antigen is termed the 'affinity'. The affinity corresponds to the sum of the attractive and repulsive forces between the antigen and the antibody in the relevant binding site of the antibody. The attractive force is determined, at least in part, by the number of attractive antigen-antibody interactions at the binding site. Non-covalent interactions, such as hydrogen bonds, electrostatic interactions, van der Waals forces and hydrophobic interactions, between the antibody and the antigen may mean that the binding of the antigen to the antibody is reversible. Accordingly, adjustment to the charge and/or configuration of the antibody may result in the number of attractive antigen-antibody interactions being reduced. Moreover, the sum of the repulsive forces may, for instance, be increased, e.g. due to net charge changes of the antibody and the antigen respectively. The 'fit' between the antigen and the antibody may therefore be disrupted by making such an adjustment, such that the antigen is released from the antibody.

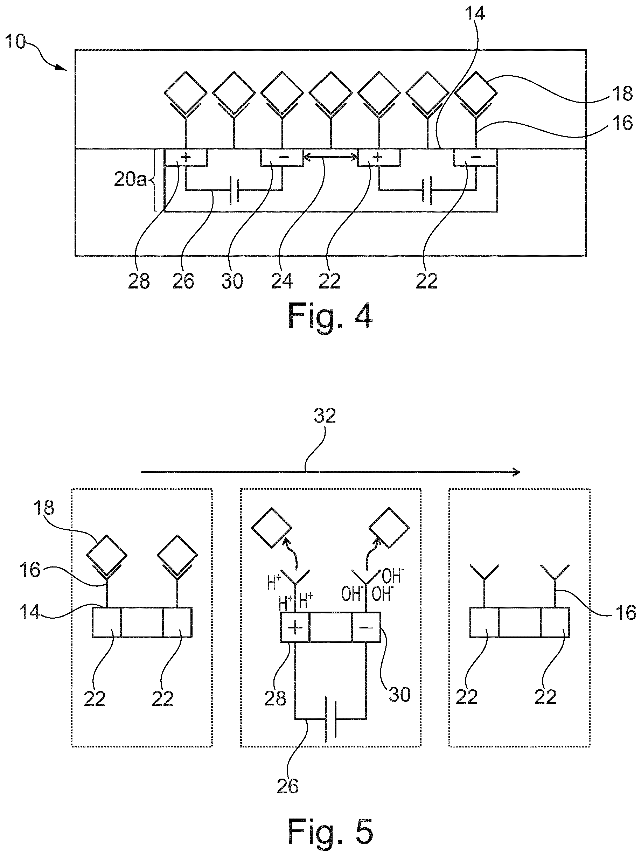

The regeneration assembly 20 is configured to regenerate the capture surface 14 by releasing the calibration molecules (i.e. the bound analyte molecules) from the capture surface 14 into an aqueous solution (e.g. buffered solution or bioliquid) to form a calibration fluid for calibrating the sensor device. Exemplary regeneration assemblies will be discussed hereafter and in particular with reference to Figs. 4 to 11.

Optionally, the aqueous solution may be a fluid sample of the subject. In other words, the calibration molecules are in the same matrix (i.e. bioliquid) as the measurement. Accordingly, the matrix effects, i.e. change of binding conditions due to interfering components in the bioliquid, may also be corrected for. A normal calibration liquid is a buffered solution and is per definition not the same liquid as the bioliquid, i.e. the fluid sample of the subject.

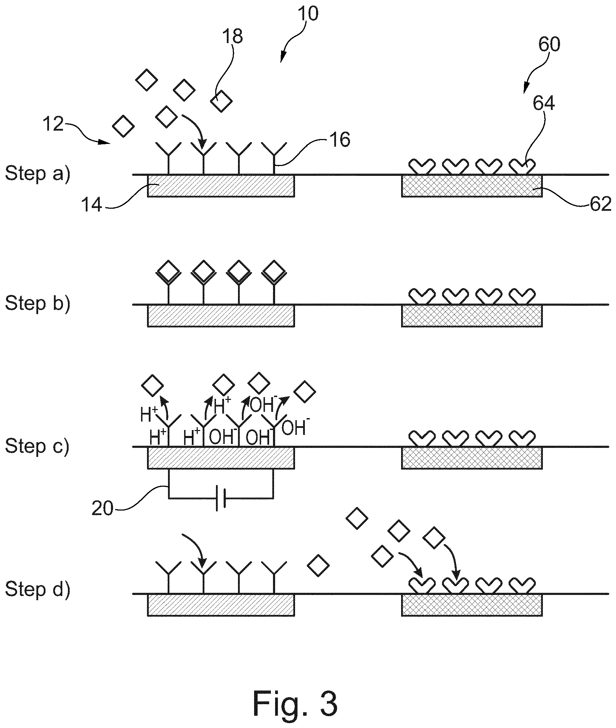

Fig. 2 illustrates a flowchart of a calibration method 300, which is described in more detail in Figs. 3A to 3D.

In step 310, i.e. step a), a calibration unit 10 receives a fluid sample of the subject. The calibration unit 10 comprises a calibration matrix 12, which has a capture surface 14 with calibration receptors 16 being immobilized thereon.

Step a) is illustrated in Fig. 3 in more detail. Note that this step comprises the exposure (for example by recirculation) of capture area 14 of the calibration unit 10 with same bioliquid as used for the sensor device 60. The sensor device 60 comprises a capture surface 62 with sensor molecules 64 being immobilized thereon. The sensor molecules 64 may be receptors for binding the analyte molecules or enzymes to facilitate the conversion of the analyte to a product. In this example, the calibration receptors 16 and the sensor molecules 64 comprise different receptors represented by different shapes. In some other examples (not shown), the calibration receptors 16 and the sensor molecules 64 may comprise same receptors.

Turning to Fig. 2, in step 320, i.e. step b), the calibration receptors 16 bind a known amount of analyte molecules 18 in the fluid sample of the subject. The bound analyte molecules represent calibration molecules usable for calibrating the sensor device.

Step b) is illustrated in Fig. 3 in more detail. In this step, a sufficient amount of molecules of interest (i.e. analyte molecule 18) is captured by the calibration receptors 16. The molecule of interest is the same molecule that is detected by the sensor device 60. The total amount of analyte molecules 18 captured by the calibration receptors 16 is driven by the Langmuir equation, which will be briefly described below.

The reaction between the analytes and the receptors can be described by the Law of Mass Action, where component A is the constant and component B is the variable:

$$[A] + [B] \xrightarrow{\frac{K_{on}}{K_{off}}} [AB] \tag{1}$$

When components A and B are mixed they form the product AB. This is called the association phase. When there is a shift to the left, component A and B are formed from product AB, which is called dissociation. The total concentration of A ($[A]_T$, where T stands for total) is split between free A and A in the AB complex. The concentration of complex [AB] is driven by the Langmuir equation:

$$[AB] = \frac{K_{on}[A]_T[B]}{K_{on}[B] + K_{off}} \left( 1 - e^{-(K_{on}[B] + K_{off})t} \right) \tag{2}$$

where $K_{on}$ is the association constant and drives the binding of the molecule of interest (B) to the receptor (A) and $K_{off}$ is the dissociation constant and drives the release of the molecule of interest (B) from the receptor (A). Note when the pH is changed the receptor changes conformation and $K_{on}$ and $K_{off}$ changes to allow for a fast release of the molecule. The reaction time (t), also referred to as incubation time, should be long enough to allow the reaction to reach equilibrium. The amount of molecules captured can therefore be controlled by choosing a predetermined amount of receptors on a certain area (concentration of A) and the type of molecule (association and dissociation) assuming the reaction is in equilibrium.

When the pre-defined amount of analyte molecules 18 is captured, it is ready to be used as a calibration solution when the conditions are met concerning equation 1. This is the time that the analyte molecule is in contact with the capture area. However, equation 1 is only valid when there is no depletion of the liquid thus the liquid needs to replenish and needs to flow over the capture surface.

Turning to Fig 2, in step 330, i.e. step c), the regeneration assembly 20 regenerates the capture surface 14 by releasing the calibration molecules from the capture surface 14 into an aqueous solution to form a calibration fluid for calibrating the sensor device.

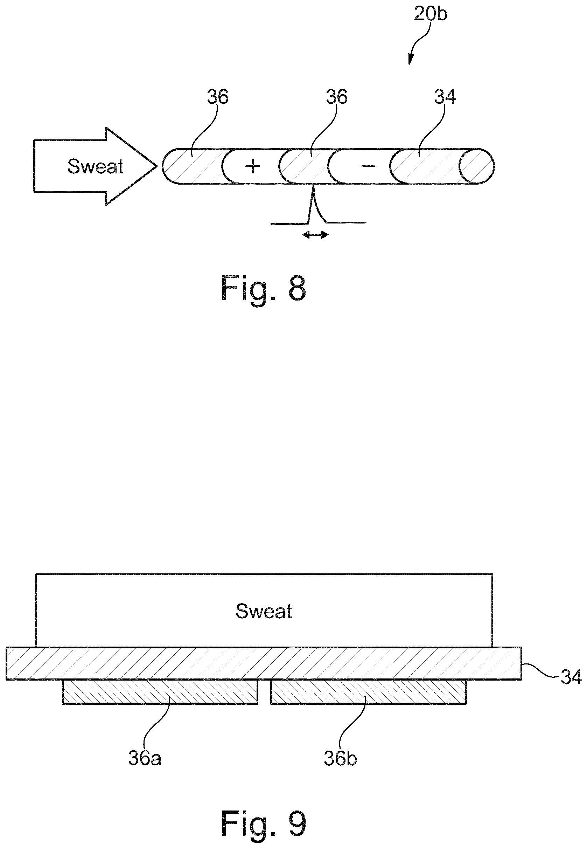

Step c) is illustrated in Fig. 3 in more detail, which illustrates the release of the calibration molecules (i.e. the bound analyte molecules) after activation of the regeneration assembly. In this example, the regeneration assembly comprises an electrode, which is activated to cause the water electrolysis. The local pH changes resulting from the water electrolysis have caused release of the bound analyte molecules 18 (i.e. calibration molecules). This example will be described in more detail with reference to Figs. 4 to 7. Further examples of the regeneration assembly will be described hereafter and in particular with reference to Figs. 8 to 11.

The release of the calibration molecules may be triggered by a need for calibration. There are several reasons for this, including: gradual chemical degradation of the sensor device, drift relating to electronic components, variation in environmental conditions, such as higher or lower temperature and humidity, changes in atmospheric pressure, exposure to relatively high concentrations of the target analyte of interest, harsh storage and operating conditions, such as when the sensor device is dropped or bumped onto a hard surface or submerged in liquid, and variation in fabrication from one sensor to another. The calibration may also be triggered by contextual and/or clinical reasons. For example, if the measurement is outside the normal reference value, it could trigger a calibration to verify that drift of the sensor is not the cause.

Turning to Fig. 2, in step 340, i.e. step d), the released calibration molecules are transported to the sensor device 60.

Step d) is illustrated in Fig. 3 in more detail. The calibration molecules in a known volume are measured by the sensor device 60. The value derived from the sensor device is used to calibrate the sensor device 60.

For example, the calibration itself may be done by comparing a known concentration of the molecules of interest (in this case coming from the 'capture area') to a signal response. The concentration sensed x(t) (i.e. concentration of the biomarker in the fluid of the patient, varying with time t) is supplemented with C, with C being a known concentration, when the captured biomarkers gets released. Therefore;

$$Calibration\ Signal = x(t) + C \tag{3}$$

x(t) is at the moment of calibration is still approximately equal to the concentration of the biomarker just before the molecules got released (i.e. $x$(t-1). Therefore, we can measure the contribution of the C to the signal.

Typically, calibration is done using a calibration curve/range. As an option, by integrating multiple capture areas and releasing them at different time intervals a calibration curve can be made to calibrate the sensor. The time separated release of calibration molecules can be done by at least one of:

(1) activating the regeneration zone at different time intervals resulting in different concentration as indicated by equation 2;

(2) by having multiple zones with different amount of receptors (i.e. size of capture area) that provide different concentrations after release; and

(3) by having receptor that release at different pH values. By incorporating a mix of these antibody types one could trigger a certain concentration by activating the same regeneration zone with different pH conditions. Note that antibodies can be designed to have different $K_{off}$ related to the pH conditions.

In the following, three examples of the regeneration assembly will be described in more detail.

The first example of the regeneration assembly is based on the realization that electrolysis of the aqueous medium may be employed to regenerate the capture surface. To this end, the regeneration assembly comprises an electrolysis assembly for electrolysing the aqueous solution, e.g. the fluid sample or the buffered solution. A voltage, e.g. a voltage profile, is supplied across pairwise combinations of a plurality of spatially separated conductive areas on the surface; the respective conductive areas of each pairwise combination thus becoming an anode and a cathode. The voltage is sufficient to electrolyse the aqueous medium, which results in hydrogen ions being produced at the anode, and hydroxyl ions being produced at the cathode. The pH local to the anode may thus be lowered, and the pH local to the cathode may be increased. These localized changes to the pH may result in disruption, e.g. denaturation, of the receptor-analyte complex, such as to regenerate at least part of the capture surface.

An exemplary electrolysis assembly 20a is illustrated in Fig. 4. The electrolysis assembly 22a comprises a plurality of spatially separated electrically conductive areas 22 on the capture surface 14. The electrically conductive areas 22 are spaced by a spacing 24. The spacing 24 may be, for instance, in the range of 0.1 mm to 3 mm, such as about 1.5 mm. The capture surface 14 patterned with the electrically conductive areas 22 may be manufactured using any suitable technique. For example, suitable patterning methodologies are known from the fields of printed electronics, thin film technology, laser ablation, etc.

The electrolysis assembly 20a may further comprises a power supply 26 for supplying a voltage across at least two of the electrically conductive areas 22; the latter thus constituting an anode 28 and a cathode 30. The voltage may be a D.C. voltage sufficient to electrolyse the water included in the aqueous solution received on the capture surface 14. Regeneration of the capture surface 14 using the electrolysis assembly 20a will be described in greater detail with reference to Fig. 5.

Fig. 5 schematically depicts regeneration of the capture surface 14 of the calibration unit 10. The capture surface 14 may become saturated when all of the calibration receptors 16 are complexed to the analyte molecules 18. The bound analyte molecules 18 represent calibration molecules.

The process of regenerating the capture surface 14 is depicted from left to right in Fig. 5, in the direction of the arrow 32. Electrically conductive areas 22 are provided on the surface 14, which conductive areas 22 constitute electrodes 28, 30 when a power supply 26 supplies a voltage across the conductive areas 22. This D.C. voltage is sufficient to electrolyse the water included in the aqueous medium of the sample when the sample contacts the capture surface 14.

In the case of a sweat sample, for example, the aqueous medium may correspond to a dilute sodium chloride solution. A physiological sodium chloride concentration may be about 50 mmol/l in sweat (cf. 140 mmol/l in plasma). A typical sodium chloride concentration used in buffers is 0.9 wt.%. Electrolysis of such a dilute sodium chloride solution may approximate the electrolysis of water: oxygen gas being generated at the anode 28 and hydrogen gas being generated at the cathode 30. Electrolysis of more concentration sodium chloride solutions may result in chlorine gas being generated at the anode 28, as is well-known per se. Some of the formed gases can be re-absorbed or eliminated from the system using a vent. Oxygen gas is normally not generated when another donor ion is present ($Na^+$, $Cl^-$) or the sodium chloride concentration is high. Hydrogen gas will be absorbed by the sweat or converted to nanobubbles. As formed gas bubbles may be reabsorbed or too small to interfere with any microfluidic flow, they may not interfere with the working of the sensor (especially since electrolysis is used to regenerate the sensor) - i.e. the biomarker measurement may occur a period of time after generation of the small amount of gas produced during electrolysis is reabsorbed/dissolved in the aqueous medium (sweat).

According to Nernst, water electrolysis is effected at voltages greater than about 1.23 V under standard conditions, i.e. standard temperature (273.15 K) and standard water electrolysis may result in a transient hydrogen ion ($H^+$) and hydroxyl ($OH^-$) ion concentration gradient pressure (100 kPa, 1 bar). In an embodiment, the voltage across the electrodes 28, 30 may be, for instance, in the range of 3 to 4.5 V.

As shown in the center pane of Fig. 5, water electrolysis may result in a transient hydrogen ion ($H^+$) and hydroxyl ($OH^-$) ion concentration gradient forming between the anode 28 and cathode 30. The localized acidic zone proximal to the anode 28 may result in the bound analyte molecules 18, i.e. the calibration molecules, being liberated from the calibration receptors 16 in the acidic zone. Alternatively or additionally, the localized alkaline zone proximal to the cathode 30 may result in the analyte molecules 18, i.e. the calibration molecules, being liberated from the calibration receptors 16 in the alkaline zone.

Whether the binding between the calibration receptors 16 and the bound analyte molecules 18 (i.e. calibration molecules) is disrupted by the more acidic and/or the more alkaline pH resulting from water electrolysis may depend, for instance, on the nature of the binding site. The preferred pH for denaturation of an antibody-antigen complex may be in the acidic range, e.g. in the range of pH 2 to 3, although denaturation may also be effected under alkaline conditions. The local pH change may, for example, drive conformational change in the calibration receptors 18. For example, when the calibration receptors 18 comprise antibodies, the local change in pH may trigger unfolding of the secondary or tertiary structure of the antibody proteins.

To reduce the risk of local build-up of an undesirably high analyte 18 concentration during regeneration of the capture surface 14, a gradual change in pH may be effected by gradually increasing the voltage. In alternative examples, the voltage may be applied in relatively short pulses or bursts, which may be preferable for inducing the localized pH changes for the regeneration of the capture surface 14 regeneration, providing the pulses may be implemented safely.

Once the local pH changes resulting from the water electrolysis have caused release of the bound analyte molecules 18, i.e. the calibration molecules, from the calibration receptors 16, the calibration receptors 16 remain adhered to the capture surface 14, as shown in the far right hand pane of Fig. 5. Due to the transient nature of the local pH change resulting from water electrolysis, the pH may revert to that before the electrolytic regeneration. The binding sites of the calibration receptors may thus be restored and may remain fully functional for binding further analyte molecules for another calibration event.

The formation of gases ($H_2$, $O_2$ or $Cl_2$, etc.) during electrolysis may not preclude calibration of the sensor device with sufficient accuracy and reliability. The gases, and $H_2$ in particular, may be absorbed in the aqueous solution. Alternatively, the gases, and $H_2$ in particular, may form 'nanobubbles' which do not or only minimally impact the calibration. The absorbed gas bubbles or nanobubbles may be too small to interfere with microfluidic flow within the calibration unit.

Moreover, a delay may be employed between regeneration and subsequent calibration to permit any small quantity of gas to dissolve before calibration. Alternatively or additionally, electrolysis gases may be released via the gas vent, and/or via channels or holes in the calibration unit. In a non-limiting example, such gases may be released from the calibration unit via the apertures of a hydrophobic mesh. Once the gases have escaped from the capture surface 14, any interference of such gases on calibration the sensor device may be avoided.

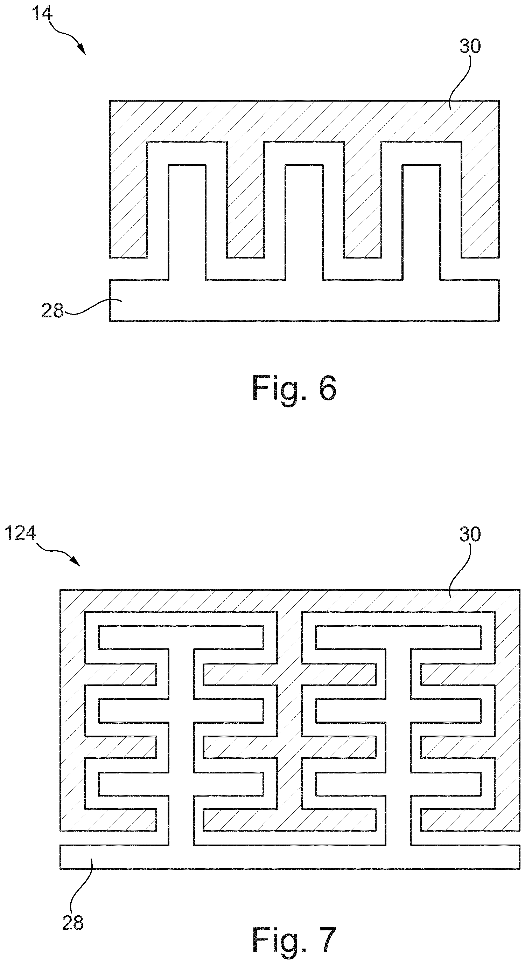

Fig. 6 shows an example of interdigitated electrically conductive areas 22. FIG. 6 shows a plan view of the capture surface 14; the calibration receptors 16 (not shown in FIG. 3) being oriented normal to the capture surface 14. When the power supply 26 provides the D.C. voltage across the electrically conductive areas 22, one of the respective conductive areas 22 becomes the anode 28 and the other of the respective conductive areas 22 becomes the cathode 30.

Fig. 7 shows interdigitated electrically conductive areas 22 according to another example. In this case, multiple interdigitated portions are provided on the capture surface 14. The comb-like interdigitated electrodes 28, 30 shown in FIGs. 6 and 7 may assist to provide the voltage over a relatively large area, e.g. in the order of several $cm^2$, whilst providing localities on the capture surface 14 in which the local pH changes resulting from water electrolysis drive regeneration of the capture surface 14 by disrupting the binding kinetics of the receptor-analyte complex.

A similar effect may be achieved using, for instance, electrically conductive areas 22 arranged as a plurality of concentric ring-like portions.

In the first example of the regeneration assembly as described above, the regeneration is accomplished by electrolysis of water molecules into H+ and OH-. This changes of pH and releases the calibration molecules for the calibration receptors.

The second example of the regeneration assembly is based on the realization that an electrical potential is generated in response to the flow of fluid through the calibration unit. The activation of the regenerable capture surface of the calibration unit is done using an internal process after the constraints resulting from the "incubation time" are met (i.e. when the condition is met concerning equation 1). In this example, the transient or pulsatile sweat flow wave (moving ions) through the microfluidic system generates a liquid triboelectric or electrochemical potential, which can be used to trigger the release of the calibration molecules. In other words, it is proposed to generate a liquid triboelectric or electrochemical potential, which is used as a passive trigger of calibration molecules release. The term "passive" in this context means that the calibration unit does not actively apply a voltage or actively change pH in order to regenerate the capture surface. Rather, it is the transient or pulsatile sweat flow wave (moving ions) through the microfluidic system that generates a liquid triboelectric or electrochemical potential.

To this end, the regeneration assembly may comprise: a flow channel arranged such that fluid flows through the flow channel; and an induction electrode (or array) arranged circumferentially around the periphery of the flow channel. The induction electrode (or array) may generate a triboelectric potential due to moving ions in the fluid flowing through the flow channel. The triboelectric potential generated by the induction electrode may correspond to the rate of moving ions in the fluid flowing through the flow channel. More specifically, to the velocity and concentration (i.e. flow rate and concentration rate) of moving ions in the fluid flowing through the flow channel. The ions moving through the flow channel may interact with the induction electrode to induce a triboelectric potential (voltage), which may be used directly as the current for the regeneration capture surface. Alternatively or additionally, the calibration unit may comprise: a flow channel arranged such that fluid flows through the flow channel; and one or more ion-selective electrodes arranged inside the flow channel. The ion-selective electrode may generate an electrochemical potential due to moving ions in the fluid flowing through the flow channel. The electrochemical potential generated by the ion-selective electrode may correspond to the rate or concentration of (specific) moving ions in the fluid flowing through the flow channel. The ions moving through the flow channel may interact with the ion-selective electrode to induce an electrochemical potential (voltage), which may be used directly as the current for the regeneration capture surface.

Using the electrodes in the calibration unit, once new fluids are generated, the (initially) transient or pulsatile fluid flow/pressure wave (moving ions) through the microfluidic system generates a liquid triboelectric or electrochemical potential (transient signal). The produced signal is harvested by the induction electrodes circumferentially placed around or ion-selective electrodes inside the flow channel of the microfluidic system, and may be used for the passive trigger of calibration molecule release.

Fig. 8 shows a diagram of the electrodes and flow channel according to the second example of the regeneration assembly. The regeneration assembly 20b comprises a flow channel 34 and an electrode arrangement 36 comprising electrodes that are either induction electrodes circumferentially arranged outside the channel 34, or ion-selective electrodes inside the channel 34. Fluids derived from skin of the user (e.g. sweat excreted by the skin, interstitial fluid pulled from the skin) flows into the flow channel 34. Fluids may be considered as moving ions in water and the ions interact with the electrode arrangement to generate a potential. The fluids may include, for example, sodium ions Na+, hydroxide ions OH-, chloride ions Cl- and/or hydrogen ions H+.

Fig. 9 shows a cross-section of exemplary induction electrodes. The electrode arrangement 36 comprise a first induction electrode 36a and a second induction electrode 36b, with a fluid channel 34 provided in cooperation with the induction electrodes. The first induction electrode 36a and the second induction electrode 36b may be formed of copper. A cross-section of the fluid channel 34 is shown in Fig. 8, which may be formed from silicone, PTFE or the like. Fluids (e.g. sweat) may be provided across the first induction electrode 36a and the second induction electrode 36b, with the fluids comprising moving ions. A triboelectric potential is thus generated through the moving ions. A 'wake-up' signal would suggest that the signal first goes to a 'processor' that processes the signal in order to use a different power supply for the regeneration

Alternatively, the liquid triboelectric or electrochemical potential may be used as a wake-up signal to trigger the activation of a regeneration assembly. For example, the wake-up signal may be supplied to a controller controlling a power supply of the regeneration assembly (e.g. power supply 26 in Fig. 4) so as to activate the controller and wake the regeneration assembly. In a wake up system with triboelectric charge induction electrodes, the voltage difference between the electrode pair is measured by an amplifier having a very large input impedance (for example, 200 T$\Omega$), as the triboelectric signal typically has a high optimum output impedance which needs to be matched and converted to a suitable wake-up current using power conversion electronics. This signal is typically an alternating signal (AC) which may need to be rectified before being utilised by the wake-up electronics.

A notable advantage associated with the second example is that no power is required to be supplied to the capture surface of the calibration unit to regenerate the capture surface.

The third example of the regeneration assembly is based on the realization that that the pH of the first fraction of fluid droplets (e.g. sweat, sebum, and interstitial fluid) excreted on the skin surface is initially around 2, which is sufficiently acidic to support release of the calibration molecules and thereafter, as the fluid rate increases, the pH rises gradually until it reaches a near neutral pH (i.e., pH ~7).

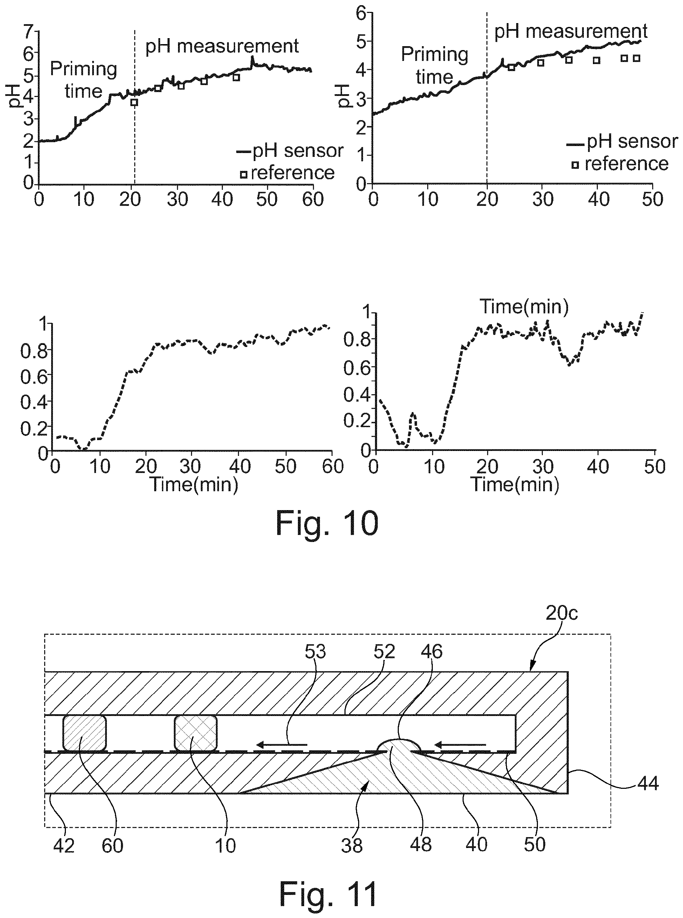

In the case of a sweat sample, for example, Fig. 10 shows sweat rate and pH values during a 60-minute effort at self-selected pace (Coyle et al, Proceedings of the 3d International ICST Conference on Pervasive Computing Technologies for Healthcare, pp. 4-9, ICST, 2009). This occurs due to the inverse relation between the reabsorption flux of ions along the sweat duct and the sweat rate (Sonner et al., Biomicrofluidics 9, 031301 2015). It is therefore important to emphasize here that only the first fraction of fluid droplet(s), such as sweat droplets, emerging on the skin surface from an active sweat gland will have a pH of 2 and only this first fraction is used to release the calibration molecules. If we assume, that the first fraction of sweat emerges on the skin surface in a hemispherical droplet ($V = \frac{2}{3}\pi r^3$ ), with radius 30-50 $\mu$m, at an average sweat rate of 0.2 nl/min/gland and during burst a sweat rate of 1.2 nl/min/gland (sweat glands operate in burst fashion with a typical cycle of: 30 seconds producing sweat and 150 seconds not producing sweat) with 10 active glands per $cm^2$, it is possible to estimate the volume of sweat with pH 2 available and the time taken for droplet formation. The sweat droplet volume, V, is:

$$V_{30} = \frac{2}{3}\pi(30 \times 10^{-6})^3 = 0.0565 \, nl$$

$$V_{50} = \frac{2}{3}\pi(50 \times 10^{-6})^3 = 0.262 \, nl$$

Furthermore, the time, t, taken for droplet formation (per gland) can be expressed in terms of volume, V, and sweat rate, Q, as:

$$t_{30} = \frac{V}{Q} = \frac{0.0565 \, nl}{1.2 \, nl/min} = 0.0471 min = 2.8 \, seconds$$

$$t_{50} = \frac{V}{Q} = \frac{0.262 \, nl}{1.2 \, nl/min} = 0.218 \, min = 13.1 \, seconds$$

These times for sweat droplet formation are reasonable and are feasible in practice.

In the following, it is proposed to transport droplets of fluids to the calibration capture surface e.g. by means of an electromechanical (e.g. electrowetting) or chemical gradient, by utilizing a method for sweat droplet. The first fraction of fluid droplet(s), such as sweat droplets, emerging on the skin surface is used to release the calibration molecules to form a calibration fluid.

Fig. 11 schematically depicts, in cross-sectional view, an exemplary regeneration assembly 20c according to the third example. This exemplary regeneration assembly is in fact a fluid collection assembly for supplying the fluid sample to the calibration unit and the sensor device. The fluid collection assembly is particularly configured for droplet discretization, e.g. sweat droplet discretization, and therefore allows the first fraction (i.e. one or more droplets) of sweat emerging on the skin to be transported to the calibration unit. This first fraction has a pH of 2 and is thus sufficiently acid to support release of the calibration molecules. Accordingly, when calibration is needed, the fluid collection assembly is used to collect a first fraction of sweat droplets excreted on the skin surface, which supports release of calibration molecules to for a calibration fluid.

The regeneration assembly 20c comprises a chamber 38 having an inlet 40. The inlet 40 receives fluid, such as sweat, from the skin 42. As shown in Fig. 11, the inlet 40 may be disposed adjacent to a surface of the skin 42. Whilst a single chamber 38 is depicted in Fig. 11, this is not intended to be limiting, and in other examples a plurality of chambers 38 may be included in the regeneration assembly 42.

To facilitate understanding of the device and method described herein, sweat will be described henceforth. The sweat excreted by a sweat gland enters and fills the chamber 38 via the inlet 40. As shown in Fig. 11, the regeneration assembly 20c may comprise a plate 44 which is attached to the surface of the skin 42. In the depicted example, a lower surface of the plate 44 is in direct contact with the surface of the skin 42. In this case, the chamber 38 takes the form of an aperture delimited by the plate 44. The plate 44 may be formed of any suitable material, e.g. a polymer, capable of being disposed on the skin. For example, the plate 44 may have at least a degree of flexibility so as to enable conformal application to the surface of the skin 42. More rigid plates 44 may also be contemplated, providing the inlet 40 can receive sweat from the skin 42.

In order to collect sweat from a subject, the plate 44 may, for instance, be adhered to the surface of the skin 42 using a suitable biocompatible adhesive. Alternatively, the plate 44 may be held against the surface of the skin 42 by fastenings, e.g. straps, for attaching the plate 44 to the body of the subject.

It is preferable that the diameter of the inlet 40 for receiving sweat from the skin 42 is selected to be relatively small, for example 200-2000 $\mu$m, such as 300-1200 $\mu$m, e.g. about 360 $\mu$m or about 1130 $\mu$m. The diameter of sweat gland outlets on the surface of the skin 42 are typically in the range of about 60 $\mu$m to 120 $\mu$m. A relatively small inlet 40 may assist to reduce the chances of two or more sweat glands excreting into the same inlet 40, which can complicate interpretation of sensor signals. To compensate for the limited amounts of sweat being received into an individual chamber 38, the regeneration assembly 20c may, for instance, include a plurality of such chambers 38, for example 2 to 50 chambers 38, such as 10 to 40 chambers 38, e.g. about 25 chambers 38.

Once the chamber 38 has been filled with sweat, a sweat droplet 46 protrudes from an outlet 48 of the chamber 38. In the example shown in Fig. 11, the outlet 48 is delimited by an upper surface of the plate 44, and a hemispherical sweat droplet 46 forms on top of the outlet 48 once the chamber 38 has been filled with sweat.

More generally, the regeneration assembly 20c may be configured such that the speed of formation of the sweat droplet 46 is determined by the sweat rate, while the volume of the sweat droplet 46 is determined by the fluid transport assembly.

The respective areas of the inlet 40 and the outlet 48 may be selected to ensure efficient filling of the chamber 38 and sweat droplet 46 formation over a range of sweat rates. In some examples, the inlet 40 and the outlet 48 have selected fixed dimensions for this purpose. Alternatively, the regeneration assembly 20c may be configurable such that at least some of the dimensions and geometry relevant to sweat droplet 46 formation can be varied.

In a preferred example (not shown in Fig. 11), the chamber 38 is dimensioned to fill up with sweat within 10-15 minutes. The formation of the hemispherical sweat droplet 46 following filling of the chamber 38 preferably occurs typically within 10 seconds at relatively low sweat rate, e.g. 0.2 nl/min/gland.

The diameter of the outlet 48 may, for example, be in the range of 10 $\mu$m to 100 $\mu$m, e.g. 15 $\mu$m to 60 $\mu$m, such as about 33 $\mu$m, in order to assist in controlling the sweat droplet size so that its volume is uniform and reproducible. By the outlet 48 having such a diameter, e.g. about 33 $\mu$m, several sweat droplets 46 may be formed during a single sweat burst (typically lasting 30 seconds) of a sweat gland, even with sweat rates as low as 0.2 nl/min/gland. Consequently, sufficient sweat droplets 46 may be generated and transported by the regeneration assembly 20c to the calibration unit 10 in order for the sweat rate to be reliably estimated.

The regeneration assembly 20c may enable the formation of relatively uniformly sized sweat droplets 46, and in addition may handle variable sweat droplet 46 volumes as well. Regarding the latter, the calibration unit to which the regeneration assembly 20c transports the sweat droplets 46 may be configured to both count the sweat droplets 46 and determine the time it takes for each sweat droplet 46 to pass through the calibration unit 10. This time is linearly related via the *a priori* known migration speed to the volume of the sweat droplet 46.

As an indication of the scale of the part of the exemplary regeneration assembly 20c shown in Fig. 11, the dimensions of the chamber 38, inlet 40, and outlet 48 may be selected according to, for instance, the sweat rate of the subject. The volume of the chamber 38 may be minimized in order to decrease the filling time. This may assist to ensure a minimal delay between actual sweat excretion and sensing/monitoring of the sweat droplets 46. For example, the volume of the chamber 38 may be in the range of 0.1-100 nl, such as 0.5-50 nl, e.g. 1-20 nl.

The volume of the chamber 38 may be minimized in various ways in order to minimize the time required to fill the chamber 38 with sweat. Such modifications may be, for instance, to the plate 44 delimiting the chamber 38.

Fig. 11 shows an example in which the chamber 38 tapers from the inlet 40 towards the outlet 48. By illustration, the length dimension of the plate 44 shown in Fig. 11 is about 500 $\mu$m. The tapering chamber 38 in this example has a conical geometry, i.e. having a truncated cone shape with a volume of $1/3\pi h[R^2 + Rr + r^2]$ (h = 50 $\mu$m; R = 360 $\mu$m; r = 33 $\mu$m). For a relatively low sweat rate of 0.2 nl/min/gland, the filling time of this tapering chamber 38 may be about 10 minutes and the sweat droplet 46 formation may take around 12 seconds. By contrast, filling of a cylindrical chamber 38 shown in Fig. 1 having the same height (50 $\mu$m) and base (360 $\mu$m) dimensions may take around 50 minutes, and the formation time of the hemispherical sweat droplet 46 may be more than 3 hours.

At this point it is noted that sweat glands tend to excrete in sweat bursts, each sweat burst being followed by a rest period in which the glands are not excreting. During the sweat burst period the sweat rate may be about six times larger than the average sweat rate. The reason is that in a time window of 180 seconds there is typically a sweat burst of 30 seconds and a rest period of typically 150 seconds, hence there is a factor of six between the average sweat rate and the sweat rate during a sweat burst. In the above illustrative example of a chamber 38 having a truncated conical shape, the time to form the depicted sweat droplet 46 is about 12 seconds during the sweat burst of the sweat gland.

In the example shown in Fig. 11, 56% of the surface area of the sweat droplet 46 is in contact with the upper surface of the plate 44. The upper surface of the plate 44 may be provided with a gradient, such as a topological and/or chemical gradient, for the purpose of releasing the sweat droplet 46 from the outlet 48 and transporting the sweat droplet 46 to the sensor, as will be discussed further herein below in relation to the fluid transport assembly. Suffice to say at this point that, in the case of such a topological and/or chemical gradient, the surface area of the sweat droplet 46, in contact with the upper surface of the plate 44, required for releasing the sweat droplet 46 from the outlet 48 may depend on the steepness of the chemical and/or topological gradient, and the volume of the sweat droplet 46.

Alternatively, the chamber 38 may be a cylindrical chamber (not shown) having the same height and base diameter dimensions.

Further, the regeneration assembly 22c comprises a fluid transport assembly which is arranged to enable release of the sweat droplet 46 protruding from the outlet 48. The fluid transport assembly may thus, for example, comprise a structure which detaches the sweat droplet 46, e.g. the hemispherical sweat droplet 46, from the outlet 48.

A formed sweat droplet 46 may be anchored to the bulk of sweat which has filled the chamber 38 due to the attractive intermolecular forces between the water molecules in the sweat.

In practice, the sweat droplet 46 does not have a single contact angle value, but rather a range from a maximum to a minimum contact angle, which are called the advancing contact angle and the receding contact angle, respectively. The difference between the advancing and receding contact angles is known as contact angle hysteresis.

These forces resist movement of the sweat droplet 46 from the outlet 48. Such forces lead to retention of the sweat droplet 46 above the filled chamber 38. The fluid transport assembly enables these forces to be overcome, such as to detach the sweat droplet 46 (and transport the sweat droplet 46 downstream towards the sensor). The fluid transport assembly may be configured to enable a well-defined dislodgement of the sweat droplet 46 from the chamber 38. In other words, detachment of the sweat droplet 46 ensures unambiguous discrete sweat droplet 46 definition.

The fluid transport assembly may, for instance, be provided with a passive and/or an active gradient for dislodging, i.e. releasing, the sweat droplet 46. The passive gradient may include a chemical and/or a topological gradient. The active gradient may be provided by an applied pressure and/or by an electric field of an electrowetting arrangement.

The detachment or release of the sweat droplet 46 may in some examples occur at the moment that the sweat droplet 46 reaches a certain diameter. At that diameter, an active and/or a passive gradient, e.g. which may be experienced by at least part of, and preferably the entirety of, the sweat droplet 46, may be sufficiently large to overcome the contact angle hysteresis of the sweat droplet 46, such that the sweat droplet 46 is released from the outlet 48.

Fig. 11 schematically depicts an example in which sweat droplet 46 detachment is effected by an electrowetting technique. This may be regarded as an example of an "active" interfacial tension method, in which a force is actively applied in order to overcome the contact angle hysteresis of the sweat droplet 46.

As shown in Fig. 11, the upper surface of the plate 44 is provided with a series of discrete electrowetting tiles 50. For the purpose of detaching from the outlet 48 and/or transporting an aqueous sweat droplet, the electrowetting tiles 50 may comprise an electrode which is coated with a hydrophobic material, such as a fluoropolymer. The transport assembly may comprise an electric field generator (not shown) for charging and discharging each of the electrowetting tiles 50 of the series in sequence. Charging of an electrowetting tile 50 may cause the surface properties of the electrowetting tile 50 to switch from hydrophobic to hydrophilic, thereby to instantaneously overcome the contact angle hysteresis of the sweat droplet 46. The sweat droplet 46 may correspondingly migrate onto the charged electrowetting tile 50. Subsequent discharge of the charged electrowetting tile 50 and charging of the subsequent electrowetting tile 50 in the series may cause the sweat droplet 46 to migrate to the subsequent electrowetting tile 50, and so on. This sequence may be regarded as an "electrowetting wave" causing the sweat droplets 46 to move to the calibration unit in a direction 53.

In the example shown in Fig. 11, detachment from the outlet 48 may occur when the sweat droplet 46 has grown to acquire a sufficiently large diameter that the sweat droplet 46 at least partially overlaps a pair of electrowetting tiles 50. In this case, once an electrowetting wave passes along the electrowetting tiles 50, the sweat droplet 46 spanning the pair of electrowetting tiles 50 will be dislodged from the outlet 48 accordingly. In this example, the sweat droplets 46 may not be all of a uniform size or volume, because the sweat droplet 46 may continue to grow to varying degrees in the period between the sweat droplet 46 reaching the requisite diameter and the arrival of the electrowetting wave. In this respect, the sweat droplet size may be determined by the frequency of the electrowetting wave.

In an alternative example (not shown), the fluid transport assembly may employ a "passive" gradient to release the sweat droplet 46 from the outlet 48. The term "passive" in this context means, in general terms, that the fluid transport assembly does not actively apply a force in order to overcome the contact angle hysteresis of the sweat droplet 46.

For instance (not shown), the upper surface of the plate 44 may be provided with a chemical and/or topological gradient which enables detachment of the sweat droplet 46 from the outlet 48. The topological gradient may be provided by the upper surface of the plate 44 being inclined, such that, when the regeneration assembly 20c is orientated for use, the gradient of the incline spanning the sweat droplet 46 diameter is sufficiently large to overcome the contact angle hysteresis. The chemical gradient may be provided by the surface having hydrophilic and hydrophobic moieties thereon, which moieties are arranged to provide a wettability gradient along the surface. For example, microfluidic channels functionalized with hydrophobic $CH_3$- moieties (towards the skin 106) and hydrophilic OH-moieties (towards the sensor) may be used to create a chemical gradient (Morgenthaler et al., Langmuir; 2003; 19(25) pp 10459-10462).

The chemical gradient may be, for example, provided with hydrophilic/hydrophobic domains at the molecular level, such that the wettability gradient varies substantially continuously along the surface. Such a chemical gradient may, for instance, be provided by grafted polymer chains functionalizing the surface of the plate 44. Alternatively or additionally, hydrophilic/hydrophobic domains of $\mu$m dimensions may be provided on the surface such as to provide a stepwise wettability gradient. Preferably, the domains are arranged to have a gradual change in distribution over the length of the surface in the direction of the sensor.

When such a passive, e.g. chemical and/or topological, gradient is employed for detachment of the sweat droplet 46, detachment may occur when the sweat droplet 46, e.g. the hemispherical sweat droplet 46, reaches a certain size. Once the diameter of the sweat droplet 46 is such that the gradient spanning the diameter is sufficiently large to overcome the contact angle hysteresis, the sweat droplet 46 will become detached from the outlet 48. In this sense, such a gradient may result in each of the sweat droplets 46 being transported to the sensor having a similar size/volume relative to each other. After the sweat droplet 46 is detached, viscous drag may also play a role in retarding sweat droplet 46 motion due to the driving force created by the surface energy gradient.

In the example shown in Fig. 11, the fluid transport assembly comprises a further plate 52 which is separated from and opposes the plate 44 delimiting the chamber 38. The further plate 52 may enable control to be exerted over the volume of the sweat droplet 46. This may be achieved, for instance, by the further plate 52 being separated from the plate 44 by a defined distance 54. The sweat droplet 46 may increase in size until it makes contact with the further plate 52. In practice, when the sweat droplet 46 contacts the further plate 52, the sweat droplet 46 may become detached by "jumping" over to the further plate 52. This may be regarded as a further example of an interfacial tension method for detaching the sweat droplet 46 from the outlet 48.

In this example, the calibration unit 10 may be pre-loaded with e.g. sweat droplets such that the capture surface 14 of the calibration unit 10 captures a known amount of analyte molecules 18 that represent calibration molecules. The first fraction of sweat droplet(s) emerging on the skin surface is used to release the molecules to form a calibration fluid, as the pH of the first fraction of sweat droplet(s) is around 2. The calibration fluid will be delivered to the sensor device 60 for calibrating the sensor device 60.

A notable advantage associated with the third example is that no power is required to be supplied to the capture surface of the calibration unit to regenerate the capture surface.

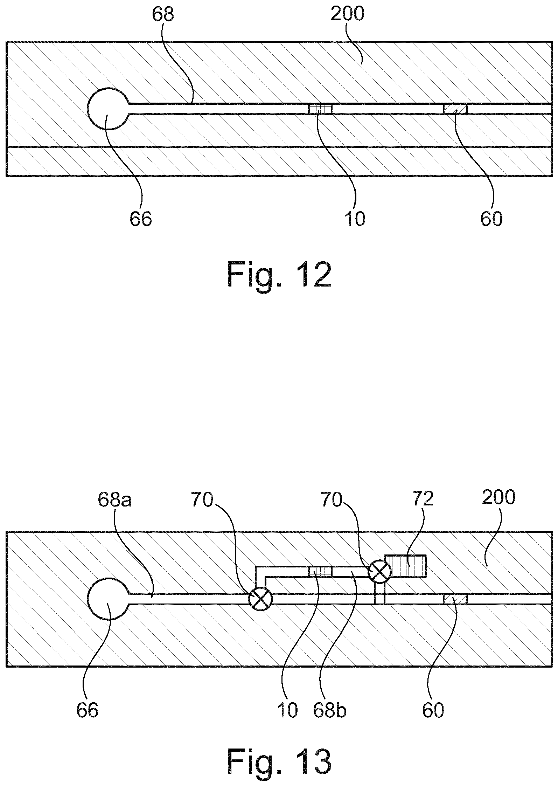

Fig. 12 schematically shows an example of a body fluid monitoring apparatus 200 for detecting an analyte in a fluid sample of a subject. In some examples, the body fluid monitoring apparatus 200 may detect body fluids (e.g. sweat, sebum and interstitial fluid) derived from the skin of the user and may be associated with the continuous or semi-continuous monitoring of the user, in particular, the fluid of a user and/or properties of the fluid of the user. In some examples, the body fluid monitoring apparatus 200 may detect other body fluids, such as blood, urine, and saliva. Examples of the body fluid monitoring apparatus may include, e.g. wearables, patches, insertables, implantables. For example, the body fluid monitoring apparatus 200 may be a wearable patch on the skin that measures sweat, an implantable device in interstitial fluid or blood, a toothbrush that measures saliva, a baby bottle that measures milk, a breast pump that measures milk, a toilet sensor that measures urine, a blood measurement outside the body, etc.

The body fluid monitoring apparatus 200 comprises a calibration unit 10 according to any example as described above.

The body fluid monitoring apparatus 200 further comprises a sensor device 60 having a capture surface 62 with sensor molecules 64 being immobilized thereon for detecting the analyte in the fluid sample. In some examples, the sensor molecules 64 may be receptors for binding the analyte molecules. In some examples, the sensor molecules may be e.g. enzymes to facilitate the conversion of the analyte to a product. The conversion gives a measurable signal. An exemplary sensor device is illustrated in Fig. 3. The sensor device 60 is in fluid communication with the calibration unit 10 at least in a calibration event.

The body fluid monitoring apparatus 200 further comprises a fluid collection assembly 66 for supplying the fluid sample to the calibration unit 10 and the sensor device 60.

In this example, the calibration unit 10 and the sensor device 60 are arranged in the same transport channel 68. Calibration molecules are bound to the capture area of the calibration unit 10. The bound calibration molecules may not preclude detection of the sensor device 60 with sufficient accuracy and reliability during body fluid monitoring. The release of the calibration molecules is triggered by a need for calibration. This can be due to time since last calibration (i.e. drift) and/or an unusually measurement value and/or an increase in background signal. The released calibration molecules are then transported to the sensor device 60, which measures the calibration molecules in a known volume. The value derived from the sensor device 60 is used to calibrate the sensor device 60.

In this example, because the capture surface of the calibration unit 10 is in the transport channel as the sensor device 60, the stability of the calibration receptors may also affected by the contact with the bioliquid. However, in the case of an electrochemical sensor, the degradation degree between the enzyme on the sensor device and the antibody on the calibration unit is different. Whereby the stability of the antibody has been proven to be much more stable. Therefore, the ability of capturing the calibration molecules to provide a constant amount of calibration molecules thought the lifetime of the wearable/patch is provided. In addition, any fouling mechanisms will be counteracted by the same elution process that is used for the regeneration process. This ensures a stable amount of calibration molecules that are captured and released by the capture surface.

Fig. 13 schematically shows another example of a body fluid monitoring apparatus 200.

In this example, the body fluid monitoring apparatus 200 comprises two transport channels 68a and 68b. The calibration unit 10 is arrange inside the transport channel 68b, whilst the sensor device is arranged in the transport channel 68b. A valve arrangement 70 is configured to control the flow of the fluid between two transport channels.

This example is designed for a prolonged time one could benefit from reducing the residence time of the capture area of the calibration unit 10 within the bioliquid. In particular, the calibration unit 10 is only loaded when the valve arrangement 70 is configured to allow for transport the fluid to the transport channel 68b. After sufficient loading time, the capture surface of the calibration unit 10 is dried with a capillary pump and evaporator arrangement 72 such that the calibration molecules bound on the capture surface of the calibration unit 10 are less prone to degenerated. When calibration is needed, the valve arrangement and the regeneration assembly are configured to release the calibration molecules and transport them to the sensor device 60.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

**Claims**

1. A calibration unit (10) adapted for calibrating a sensor device (60) adapted for detecting analyte molecules (18) in a fluid sample of a subject, the calibration unit comprising:

   - a calibration matrix (12) having a capture surface (14) with calibration receptors (16) being immobilized thereon adapted for reversibly binding a known amount of the analyte molecules (18) in a fluid sample of the subject, wherein the bound analyte molecules represent calibration molecules usable for calibrating the sensor device; and
   - a regeneration assembly (20, 20a, 20b, 20c) configured to regenerate the capture surface (14) by releasing the calibration molecules from the capture surface into an aqueous solution to form a calibration fluid for calibrating the sensor device.

2. Calibration unit according to claim 1,
   wherein the aqueous solution is a fluid sample of the subject.

3. Calibration unit according to claim 1 or 2,
   wherein the regeneration assembly is configured to apply an electrical potential to the capture surface to release the calibration molecules from the capture surface.

4. Calibration unit according to any one of the preceding claims,
   wherein the regeneration assembly comprises an electrolysis assembly (20c) configured to electrolyse the aqueous solution.

5. Calibration unit according to claim 4,
   wherein the electrolysis assembly comprises:

   - at least three spatially separated electrically conductive areas (22) on the capture surface; and
   - a power supply configured to implement:

     - a first setting in which a voltage sufficient to electrolyse the aqueous solution received on the capture surface is supplied across a first pairwise combination of said at least three conductive areas; and
     - a second setting in which a voltage sufficient to electrolyse the aqueous solution received on the capture surface is supplied across a second pairwise combination of said at least three conductive areas, the second pairwise combination being different from said first pairwise combination.

6. Calibration unit according to any one of the preceding claims, further comprising:

   - an assembly (20b) that comprises:

     - a flow channel (34) arranged such that the aqueous solution flows through the flow channel; and
     - an electrode arrangement (36) comprising at least one of an induction electrode and an ion-selective electrode;
     wherein the induction electrode is arranged circumferentially around a periphery of the flow channel and configured to generate a triboelectric potential in response to moving ions in the aqueous solution flowing through the flow channel; and
     wherein the ion-selective electrode is arranged inside the flow channel and configured to generate an electrochemical potential in response to moving ions in the aqueous solution flowing through the flow channel.

7. Calibration unit according to claim 6,
wherein the assembly is (i) a regeneration assembly configured to apply at least one of the triboelectric potential and the electrochemical potential to the capture surface to release the calibration molecules from the capture surface; or (ii) a trigger assembly configured to trigger the activation of the regeneration assembly when at least one of the generated triboelectric potential and the generated electrochemical potential exceeds a predetermined threshold corresponding to a given rate of moving ions.

8. Calibration unit according to claim 1 or 2,

wherein the regeneration assembly comprises a fluid collection assembly (20c) that comprises:

- a chamber (38) having an inlet (40) for receiving a droplet (46) of a fluid sample excreted on a skin surface, and an outlet (48) arranged such that the droplet of the fluid sample forms and protrudes therefrom following filling of the chamber with the fluid sample;
- a fluid transport assembly (50) configured to release the droplet protruding from the outlet and transport the released droplet to the calibration matrix, thereby making the outlet available for a subsequent droplet to form and protrude therefrom upon further filing of the chamber;

wherein the fluid transport assembly is arranged to transport the released droplet at least as fast as the subsequent droplet protrudes from the outlet such that the respective droplets do not contact each other; and
wherein the fluid transport assembly is configured to transport one or more droplets initially excreted on the skin surface to the calibration matrix to release the calibration molecules from the capture surface to form the calibration fluid.

9. Calibration unit according to any one of the preceding claims,
wherein the calibration matrix is configured to release a different amount of calibration molecules into the aqueous solution to form calibration fluids with different concentrations to construct a calibration curve to calibrate the sensor device.

10. Calibration unit according to claim 9,
wherein calibration fluids with different concentrations are formed by at least one of:

- activating the regeneration assembly at different time intervals to release the calibration molecules from the capture surface, thereby resulting in different concentrations;
- providing multiple capture areas, each of which having a different amount of calibration receptors such that each capture area release a different amount of calibration molecules; and
- having different types of calibration receptors, each of which configured to release the calibration molecules at a different amount of hydrogen ions.

11. Calibration unit according to any one of the preceding claims, further comprising:

- a capillary pump configured to remove a fluid from the capture area; and
- an evaporator configured to evaporate the removed fluid.

12. A body fluid monitoring apparatus for detecting an analyte in a fluid sample of a subject, the body fluid monitoring apparatus comprising:

- a calibration unit (10) according to any one of the preceding claims;
- a sensor device (60) having a capture surface (62) with sensor molecules (64) being immobilized thereon for detecting the analyte in the fluid sample, wherein the sensor device is in fluid communication with the calibration unit at least in a calibration event; and
- a fluid collection assembly (66) for supplying the fluid sample to the calibration unit (10) and the sensor device (60).

13. Body fluid monitoring apparatus according to claim 12, comprising:

(i) a transport channel (68) arranged to accommodate both the calibration unit and the sensor device; or
(ii) two transport channels (68a, 68b) arranged to separately accommodate the calibration unit and the sensor device and a valve arrangement configured to control the flow of a fluid between the two transport channels.

14. An in vitro method (300) for calibrating a sensor device (60) for detecting an analyte in a fluid sample of a subject, the method comprising:

a) receiving (310), with a calibration unit (10), a fluid sample of the subject; wherein the calibration unit (10) comprises a calibration matrix (12) having a capture surface (14) with calibration receptors (16) being immobilized thereon;
b) reversibly binding (320), with the calibration receptors (16), a known amount of analyte molecules (18) in the fluid sample of the subject, wherein the bound analyte molecules represent calibration molecules usable for calibrating the sensor device (60); and
c) regenerating (330) the capture surface (14), with a regeneration assembly, by releasing the calibration molecules from the capture surface into an aqueous solution to form a calibration fluid; and
d) transporting (340) the released calibration molecules to the sensor device (60) for calibrating the sensor device.

15. A computer program element for calibrating a sensor device (60) for detecting an analyte in a fluid sample of a subject, which computer program element, when being executed by a processor, causes the processor to carry out the method according to claim 14.

**Patentansprüche**

1. Kalibrierungseinheit (10), die zum Kalibrieren einer Sensorvorrichtung (60) geeignet ist, die zum Nachweis von Analytmolekülen (18) in einer Flüssigkeitsprobe eines Subjekts geeignet ist, wobei die Kalibrierungseinheit umfasst:

- eine Kalibrierungsmatrix (12) mit einer Einfangoberfläche (14) mit darauf immobilisierten Kalibrierungsrezeptoren (16), die dazu ausgelegt ist, eine bekannte Menge der Analytmoleküle (18) in einer Flüssigkeitsprobe des Subjekts reversibel zu binden, wobei die gebundenen Analytmoleküle stellen Kalibrierungsmoleküle dar, die zum Kalibrieren der Sensorvorrichtung verwendet werden können; und
- eine Regenerationsanordnung (20, 20a, 20b, 20c), die so konfiguriert ist, dass sie die Einfangoberfläche (14) regeneriert, indem sie die Kalibrierungsmoleküle von der Einfangoberfläche in eine wässrige Lösung freisetzt, um eine Kalibrierungsflüssigkeit zum Kalibrieren der Sensorvorrichtung zu bilden.

2. Kalibriereinheit nach Anspruch 1, wobei die wässrige Lösung eine Flüssigkeitsprobe des Subjekts ist.

3. Kalibriereinheit nach Anspruch 1 oder 2, wobei die Regenerationsanordnung so konfiguriert ist, dass sie ein elektrisches Potenzial an die Einfangoberfläche anlegt, um die Kalibrierungsmoleküle von der Einfangoberfläche freizusetzen.

4. Kalibriereinheit nach einem der vorhergehenden Ansprüche, wobei die Regenerationsanordnung eine Elektrolyseanordnung (20c) umfasst, die zur Elektrolyse der wässrigen Lösung konfiguriert ist.

5. Kalibriereinheit nach Anspruch 4, wobei die Elektrolysebaugruppe Folgendes umfasst:

- mindestens drei räumlich getrennte elektrisch leitfähige Bereiche (22) auf der Einfangfläche; und
- ein Netzteil, das für die Implementierung von Folgendem konfiguriert ist:

- eine erste Einstellung, bei der eine Spannung, die ausreicht, um die auf der Einfangoberfläche aufgenommene wässrige Lösung zu elektrolysieren, über eine erste paarweise Kombination der mindestens drei leitenden Bereiche angelegt wird; und
- eine zweite Einstellung, bei der eine Spannung, die ausreicht, um die auf der Einfangoberfläche aufgenommene wässrige Lösung zu elektrolysieren, über eine zweite paarweise Kombination der mindestens drei leitenden Bereiche angelegt wird, wobei sich die zweite paarweise Kombination von der ersten paarweisen Kombination unterscheidet.

6. Kalibriereinheit nach einem der vorhergehenden Ansprüche, weiterhin umfassend:

- eine Baugruppe (20b), die umfasst:

- einen Strömungskanal (34), der so angeordnet ist, dass die wässrige Lösung durch den Strömungskanal fließt; und
- eine Elektrodenanordnung (36), die mindestens eine Induktionselektrode und eine ionenselektive Elektrode umfasst;

wobei die Induktionselektrode umlaufend um eine Peripherie des Strömungskanals angeordnet und so konfiguriert ist, dass sie als Reaktion auf sich bewegende Ionen in der wässrigen Lösung, die durch den Strömungskanal fließt, ein triboelektrisches Potential erzeugt; und wobei die ionenselektive Elektrode innerhalb des Strömungskanals angeordnet und so konfiguriert ist, dass sie als Reaktion auf sich bewegende Ionen in der wässrigen Lösung, die durch den Strömungskanal fließt, ein elektrochemisches Potential erzeugt.

7. Kalibriereinheit nach Anspruch 6, wobei die Baugruppe (i) eine Regenerationsbaugruppe ist, die so konfiguriert ist, dass sie mindestens eines von triboelektrischem Potential und elektrochemischem Potential an die Einfangoberfläche anlegt, um die Kalibrierungsmoleküle von der Einfangoberfläche freizusetzen; oder (ii) eine Auslöserbaugruppe, die so konfiguriert ist, dass sie die Aktivierung der Regenerationsbaugruppe auslöst, wenn mindestens eines von dem erzeugten triboelektrischen Potenzial und dem erzeugten elektrochemischen Potenzial einen vorgegebenen Schwellenwert überschreitet, der einer gegebenen Geschwindigkeit der sich bewegenden Ionen entspricht.

8. Kalibriereinheit nach Anspruch 1 oder 2, wobei die Regenerationsbaugruppe eine Flüssigkeitssammelbaugruppe (20c) umfasst, die Folgendes umfasst:

- eine Kammer (38) mit einem Einlass (40) zum Aufnehmen eines Tröpfchens (46) einer auf einer Hautoberfläche ausgeschiedenen Flüssigkeitsprobe und einem Auslass (48), der so angeordnet ist, dass sich das Tröpfchen der Flüssigkeitsprobe nach dem Befüllen bildet und daraus hervorsteht der Kammer mit der Flüssigkeitsprobe;
- eine Flüssigkeitstransportanordnung (50), die so konfiguriert ist, dass sie das aus dem Auslass herausragende Tröpfchen freigibt und das freigesetzte Tröpfchen zur Kalibrierungsmatrix transportiert, wodurch der Auslass für die Bildung eines nachfolgenden Tröpfchens verfügbar gemacht wird, dass bei weiterer Befüllung der Kammer aus diesem herausragt; wobei die Fluidtransportanordnung so angeordnet ist, dass sie das freigesetzte Tröpfchen mindestens so schnell transportiert, wie das nachfolgende Tröpfchen aus dem Auslass herausragt, sodass die jeweiligen Tröpfchen einander nicht berühren; und wobei die Flüssigkeitstransportanordnung so konfiguriert ist, dass sie ein oder mehrere anfänglich auf der Hautoberfläche ausgeschiedene Tröpfchen zur Kalibrierungsmatrix transportiert, um die Kalibrierungsmoleküle von der Einfangoberfläche freizusetzen und die Kalibrierungsflüssigkeit zu bilden.

9. Kalibriereinheit nach einem der vorhergehenden Ansprüche, wobei die Kalibrierungsmatrix so konfiguriert ist, dass sie eine unterschiedliche Menge an Kalibrierungsmolekülen in die wässrige Lösung abgibt, um Kalibrierungsflüssigkeiten mit unterschiedlichen Konzentrationen zu bilden, um eine Kalibrierungskurve zum Kalibrieren der Sensorvorrichtung zu erstellen.

10. Kalibriereinheit nach Anspruch 9, wobei Kalibrierflüssigkeiten mit unterschiedlichen Konzentrationen gebildet werden durch mindestens eines von:

- aktivieren der Regenerationsanordnung in unterschiedlichen Zeitintervallen, um die Kalibrierungsmoleküle von der Einfangoberfläche freizusetzen, was zu unterschiedlichen Konzentrationen führt;
- bereitstellen mehrerer Einfangbereiche, von denen jeder eine unterschiedliche Menge an Kalibrierungsrezeptoren aufweist, so dass jeder Einfangbereich eine unterschiedliche Menge an Kalibrierungsmolekülen freisetzt; und
- mit verschiedenen Arten von Kalibrierungsrezeptoren, von denen jeder so konfiguriert ist, dass er die Kalibrierungsmoleküle bei einer unterschiedlichen Menge an Wasserstoffionen freisetzt.

11. Kalibriereinheit nach einem der vorhergehenden Ansprüche, weiterhin umfassend:

- eine Kapillarpumpe, die so konfiguriert ist, dass sie eine Flüssigkeit aus dem Auffangbereich entfernt; und
- einen Verdampfer, der so konfiguriert ist, dass er die entfernte Flüssigkeit verdampft.

**12.** Vorrichtung zur Überwachung von Körperflüssigkeiten zum Nachweis eines Analyten in einer Flüssigkeitsprobe eines Subjekts, wobei die Vorrichtung zur Überwachung von Körperflüssigkeit umfasst:

- eine Kalibriereinheit (10) nach einem der vorhergehenden Ansprüche;
- eine Sensorvorrichtung (60) mit einer Einfangoberfläche (62) mit darauf immobilisierten Sensormolekülen (64) zum Nachweis des Analyten in der Fluidprobe, wobei die Sensorvorrichtung zumindest bei einem Kalibrierungsereignis in Fluidkommunikation mit der Kalibrierungseinheit steht; und
- eine Flüssigkeitssammelanordnung (66) zum Zuführen der Flüssigkeitsprobe zur Kalibriereinheit (10) und zur Sensorvorrichtung (60).

**13.** Vorrichtung zur Überwachung von Körperflüssigkeiten nach Anspruch 12, umfassend:

(i) einen Transportkanal (68), der so angeordnet ist, dass er sowohl die Kalibrierungseinheit als auch die Sensorvorrichtung aufnimmt; oder

(ii) zwei Transportkanäle (68a, 68b) die so angeordnet sind, dass sie die Kalibrierungseinheit und die Sensorvorrichtung getrennt aufnehmen, und eine Ventilanordnung, die so konfiguriert ist, dass sie den Fluss eines Fluids zwischen den beiden Transportkanälen steuert.

**14.** In-vitro-Verfahren (300) zum Kalibrieren einer Sensorvorrichtung (60) zum Nachweis eines Analyten in einer Flüssigkeitsprobe eines Subjekts, wobei das Verfahren Folgendes umfasst:

a) Empfangen (310) einer Flüssigkeitsprobe des Subjekts mit einer Kalibrierungseinheit (10); wobei die Kalibrierungseinheit (10) eine Kalibrierungsmatrix (12) mit einer Einfangoberfläche (14) umfasst, auf der Kalibrierungsrezeptoren (16) immobilisiert sind;

b) reversibles Binden (320) einer bekannten Menge an Analytmolekülen (18) in der Flüssigkeitsprobe des Subjekts mit den Kalibrierungsrezeptoren (16), wobei die gebundenen Analytmoleküle Kalibrierungsmoleküle darstellen, die zur Kalibrierung der Sensorvorrichtung (60) verwendet werden können; und

c) Regenerieren (330) der Einfangoberfläche (14) mit einer Regenerationsanordnung durch Freisetzung der Kalibrierungsmoleküle von der Einfangoberfläche in eine wässrige Lösung, um eine Kalibrierungsflüssigkeit zu bilden; und

d) Transportieren (340) der freigesetzten Kalibriermoleküle zur Sensorvorrichtung (60) zur Kalibrierung der Sensorvorrichtung.

**15.** Computerprogrammelement zum Kalibrieren einer Sensorvorrichtung (60) zum Nachweis eines Analyten in einer Flüssigkeitsprobe eines Subjekts, wobei das Computerprogrammelement bei Ausführung durch einen Prozessor den Prozessor veranlasst, das Verfahren nach Anspruch 14 auszuführen.

**Revendications**

**1.** Unité d'étalonnage (10) adaptée à l'étalonnage d'un dispositif de capteur (60) adapté à la détection de molécules d'analyte (18) dans un échantillon de fluide d'un sujet, l'unité d'étalonnage comprenant:

- une matrice d'étalonnage (12) comportant une surface de capture (14) avec des récepteurs d'étalonnage (16) immobilisés sur celle-ci, adaptés pour lier de manière réversible une quantité connue de molécules d'analyte (18) dans un échantillon de fluide du sujet, les molécules d'analyte liées représentant des molécules d'étalonnage utilisables pour l'étalonnage du dispositif de détection; et

- un ensemble de régénération (20, 20a, 20b, 20c) configuré pour régénérer la surface de capture (14) en libérant les molécules d'étalonnage de la surface de capture dans une solution aqueuse afin de former un fluide d'étalonnage pour étalonner le dispositif de détection.

**2.** Unité d'étalonnage selon la revendication 1, dans laquelle la solution aqueuse est un échantillon de fluide du sujet.

**3.** Unité d'étalonnage selon la revendication 1 ou 2, dans laquelle l'ensemble de régénération est configuré pour appliquer un potentiel électrique à la surface de capture afin de libérer les molécules d'étalonnage de la surface de capture.

**4.** Unité d'étalonnage selon l'une quelconque des revendications précédentes, dans laquelle l'ensemble de régénération comprend un ensemble d'électrolyse (20c) configuré pour électrolyser la solution aqueuse.

5. Unité de calibrage selon la revendication 4, dans laquelle l'ensemble d'électrolyse comprend:

- au moins trois zones électriquement conductrices (22) séparées dans l'espace sur la surface de capture; et
- une alimentation électrique configurée pour mettre en œuvre:

- un premier réglage dans lequel une tension suffisante pour électrolyser la solution aqueuse reçue sur la surface de capture est fournie à travers une première combinaison par paire desdites au moins trois zones conductrices; et
- un deuxième réglage dans lequel une tension suffisante pour électrolyser la solution aqueuse reçue sur la surface de capture est fournie à travers une deuxième combinaison par paire de ces au moins trois zones conductrices, la deuxième combinaison par paire étant différente de ladite première combinaison par paire.

6. Unité d'étalonnage selon l'une quelconque des revendications précédentes, comprenant en outre:

- un ensemble (20b) qui comprend:

- un canal d'écoulement (34) disposé de manière à ce que la solution aqueuse s'écoule à travers le canal d'écoulement; et
- un agencement d'électrodes (36) comprenant au moins une électrode d'induction et une électrode sélective d'ions; où l'électrode d'induction est disposée de manière circonférentielle autour d'une périphérie du canal d'écoulement et configurée pour générer un potentiel triboélectrique en réponse au déplacement des ions dans la solution aqueuse qui s'écoule dans le canal d'écoulement; et l'électrode sélective d'ions est disposée à l'intérieur du canal d'écoulement et configurée pour générer un potentiel électrochimique en réponse au déplacement des ions dans la solution aqueuse qui s'écoule dans le canal d'écoulement.

7. Unité d'étalonnage selon la revendication 6, dans laquelle l'ensemble est (i) un ensemble de régénération configuré pour appliquer au moins l'un du potentiel triboélectrique et du potentiel électrochimique à la surface de capture pour libérer les molécules d'étalonnage de la surface de capture; ou (ii) un ensemble de déclenchement configuré pour déclencher l'activation de l'ensemble de régénération lorsqu'au moins l'un du potentiel triboélectrique généré et du potentiel électrochimique généré dépasse un seuil prédéterminé correspondant à un taux donné de déplacement d'ions.

8. Unité d'étalonnage selon la revendication 1 ou 2, dans laquelle l'ensemble de régénération comprend un ensemble de collecte de fluide (20c) qui comprend:

- une chambre (38) ayant une entrée (40) pour recevoir une gouttelette (46) d'un échantillon de fluide excrété sur une surface de peau, et une sortie (48) disposée de telle sorte que la gouttelette de l'échantillon de fluide se forme et fasse saillie à partir de celle-ci après le remplissage de la chambre avec l'échantillon de fluide;
- un ensemble de transport de fluide (50) configuré pour libérer la gouttelette qui dépasse de la sortie et transporter la gouttelette libérée vers la matrice d'étalonnage, rendant ainsi la sortie disponible pour qu'une gouttelette ultérieure se forme et dépasse de celle-ci lors d'un nouveau remplissage de la chambre; dans lequel l'ensemble de transport de fluide est conçu pour transporter la gouttelette libérée au moins aussi vite que la gouttelette ultérieure dépasse de la sortie, de sorte que les gouttelettes respectives ne se touchent pas l'une avec l'autre; et dans lequel l'ensemble de transport de fluide est configuré pour transporter une ou plusieurs gouttelettes initialement excrétées sur la surface de la peau vers la matrice d'étalonnage afin de libérer les molécules d'étalonnage de la surface de capture pour former le fluide d'étalonnage.

9. Unité d'étalonnage selon l'une des revendications précédentes, dans laquelle la matrice d'étalonnage est configurée pour libérer une quantité différente de molécules d'étalonnage dans la solution aqueuse afin de former des fluides d'étalonnage avec des concentrations différentes pour construire une courbe d'étalonnage afin d'étalonner le dispositif de détection.

10. Unité d'étalonnage selon la revendication 9, dans laquelle les fluides d'étalonnage de différentes concentrations sont formés par au moins l'un des éléments suivants :

- activer l'ensemble de régénération à différents intervalles de temps pour libérer les molécules d'étalonnage de la surface de capture, ce qui permet d'obtenir différentes concentrations;
- fournir plusieurs zones de capture, chacune ayant une quantité différente de récepteurs d'étalonnage de sorte que chaque zone de capture libère une quantité différente de molécules d'étalonnage; et
- disposer de différents types de récepteurs d'étalonnage, chacun étant configuré pour libérer les molécules d'étalonnage à une quantité différente d'ions hydrogène.

11. Unité d'étalonnage selon l'une quelconque des revendications précédentes, comprenant en outre:

    - une pompe capillaire configurée pour extraire un fluide de la zone de capture; et
    - un évaporateur configuré pour évaporer le fluide prélevé.

12. Appareil de surveillance des fluides corporels pour détecter un analyte dans un échantillon de fluide d'un sujet, l'appareil de surveillance des fluides corporels comprenant:

    - une unité d'étalonnage (10) selon l'une quelconque des revendications précédentes;
    - un dispositif de détection (60) ayant une surface de capture (62) sur laquelle sont immobilisées des molécules de détection (64) pour détecter l'analyte dans l'échantillon de fluide, dans lequel le dispositif de détection est en communication fluidique avec l'unité d'étalonnage au moins lors d'un événement d'étalonnage; et
    - un ensemble de collecte de fluide (66) pour fournir l'échantillon de fluide à l'unité d'étalonnage (10) et au dispositif de détection (60).

13. Appareil de surveillance des fluides corporels selon la revendication 12, comprenant:

    (i) un canal de transport (68) conçu pour accueillir à la fois l'unité d'étalonnage et le dispositif de détection; ou
    (ii) deux canaux de transport (68a, 68b) conçus pour accueillir séparément l'unité d'étalonnage et le dispositif de détection, ainsi qu'une vanne configurée pour contrôler l'écoulement d'un fluide entre les deux canaux de transport.

14. Méthode in vitro (300) d'étalonnage d'un dispositif de détection (60) d'un analyte dans un échantillon de fluide d'un sujet, la méthode comprenant:

    a) recevoir (310), avec une unité d'étalonnage (10), un échantillon de fluide du sujet; l'unité d'étalonnage (10) comprend une matrice d'étalonnage (12) ayant une surface de capture (14) avec des récepteurs d'étalonnage (16) immobilisés sur celle-ci;
    b) lier de manière réversible (320), avec les récepteurs d'étalonnage (16), une quantité connue de molécules d'analyte (18) dans l'échantillon de fluide du sujet, les molécules d'analyte liées représentant des molécules d'étalonnage utilisables pour l'étalonnage du dispositif de détection (60); et
    c) régénérer (330) la surface de capture (14), à l'aide d'un ensemble de régénération, en libérant les molécules d'étalonnage de la surface de capture dans une solution aqueuse pour former un fluide d'étalonnage; et
    d) transporter (340) les molécules d'étalonnage libérées vers le dispositif de détection (60) pour étalonner le dispositif de détection.

15. Élément de programme d'ordinateur pour calibrer un dispositif de capteur (60) pour détecter un analyte dans un échantillon de fluide d'un sujet, lequel élément de programme d'ordinateur, lorsqu'il est exécuté par un processeur, amène le processeur à exécuter la méthode selon la revendication 14.

Fig. 1

Fig. 2

Fig. 3

Fig. 4

Fig. 5

14

30

28

Fig. 6

124

30

28

Fig. 7

20b

36    36    34

Sweat

+    −

Fig. 8

Sweat

34

36a    36b

Fig. 9

Fig. 10

Fig. 11

Fig. 12

Fig. 13

**EP 4 150 340 B1**

**REFERENCES CITED IN THE DESCRIPTION**

**Patent documents cited in the description**

- US 2019142311 A1

**Non-patent literature cited in the description**

- **MORGENTHALER et al.** *Langmuir,* 2003, vol. 19 (25), 10459-10462